# EUROPÄISCHE PATENTSCHRIFT

(11) **EP 1 670 488 B1**
(45) Veröffentlichungstag und Bekanntmachung des Hinweises auf die Patenterteilung: **22.04.2009**
(21) Anmeldenummer: 04766705.0
(22) Anmeldetag: 03.09.2004
(51) Int. Cl.: A61K 33/00, A61Q 5/00, A61Q 19/08, A61K 8/22

(54) **VERFAHREN ZUM AUFTRAG VON SAUERSTOFF AUF DIE HAUT**
PROCESS FOR THE APPLICATION OF OXYGEN TO THE SKIN
PROCEDE POUR APPLIQUER DE L'OXYGENE SUR LA PEAU

(30) Priorität: 13.09.2003 DE 10342449
(43) Veröffentlichungstag der Anmeldung: 21.06.2006
(73) Patentinhaber: Beiersdorf AG, 20245 Hamburg (DE)
(72) Erfinder: STAEB, Franz, 21379 Echem (DE); RIEDEL, Heidi, 22083 Hamburg (DE); BLECKMANN, Andreas, 22926 Ahrensburg (DE); BLATT, Thomas, 22880 Wedel (DE); MUMMERT, Christopher, 29553 Bienenbüttel (DE); KOLBE, Ludger, 21255 Dohren (DE)
(86) Internationale Anmeldenummer: PCT/EP2004/052042
(87) Internationale Veröffentlichungsnummer: WO 2005/027869

(56) Entgegenhaltungen:
- WO-A-02/00246
- WO-A-94/00109
- WO-A-03/022238
- WO-A-20/05016309
- DE-A- 10 113 048
- GB-A- 483 390
- US-A- 5 516 517
- US-A- 5 874 093
- STANZL K, ZASTROW L, RODING J, ARTMANN C: "The effectiveness of molecular oxygen in cosmetic formulations" INTERNATIONAL JOURNAL OF COSMETIC SCIENCE, Bd. 18, 1996, Seiten 137-150, XP009042673

## Beschreibung

Die vorliegende Erfindung betrifft die Verwendung von Sauerstoff in kosmetischen Zubereitungen zur Prophylaxe vor und Behandlung von Hautalterungserscheinungen, wie beispielsweise Falten und Fältchen, Haut- und Gewebeerschlaffung, Störungen in der Hautregeneration, Durchblutungsstörungen der Haut, Altersflecken und dergleichen. Die vorliegende Erfindung betrifft ferner die Verwendung von Sauerstoff in kosmetischen und dermatologischen Zubereitungen zur Stärkung der Barrierefunktion der Haut.

Unter kosmetischer Hautpflege ist in erster Linie zu verstehen, daß die natürliche Funktion der Haut als Barriere gegen Umwelteinflüsse (z. B. Schmutz, Chemikalien, Mikroorganismen) und gegen den Verlust von körpereigenen Stoffen (z. B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Bei alter Haut beispielsweise erfolgt die regenerative Erneuerung verlangsamt, wobei insbesondere das Wasserbindungsvermögen der Hornschicht nachläßt. Sie wird deshalb inflexibel, trocken und rissig ("physiologisch" trockene Haut). Ein Barriereschaden ist die Folge. Die Haut wird anfällig für negative Umwelteinflüsse wie die Invasion von Mikroorganismen, Toxinen und Allergenen. Als Folge kann es sogar zu toxischen oder allergischen Hautreaktionen kommen.

Bei pathologisch trockener und empfindlicher Haut liegt ein Barriereschaden a priori vor. Epidermale Interzellularlipide werden fehlerhaft oder in ungenügender Menge bzw. Zusammensetzung gebildet. Die Konsequenz ist eine erhöhte Durchlässigkeit der Hornschicht und ein unzureichender Schutz der Haut vor Verlust an hygroskopischen Substanzen und Wasser.

Die chronologische Hautalterung wird z. B. durch endogene, genetisch determinierte Faktoren verursacht. In Epidermis und Dermis kommt es alterungsbedingt z. B. zu folgenden Strukturschäden und Funktionsstörungen, die auch unter den Begriff "Senile Xerosis" fallen können:
a) Trockenheit, Rauhigkeit und Ausbildung von Trockenheitsfältchen,
b) Juckreiz und
c) verminderte Rückfettung durch Talgdrüsen (z. B. nach Waschen).
   Exogene Faktoren, wie UV-Licht und chemische Noxen, können kumulativ wirksam sein und z. B. die endogenen Alterungsprozesse beschleunigen bzw. sie ergänzen. In Epidermis und Dermis kommt es insbesondere durch exogene Faktoren z. B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z. B. Altersflecken) und
g) vergrößerte Anfälligkeit gegenüber mechanischem Stress (z. B. Rissigkeit).

Die vorliegende Erfindung betrifft insbesondere Produkte zur Pflege der auf natürliche Weise gealterten Haut, sowie zur Behandlung der Folgeschäden der Lichtalterung, ins besondere der unter a) bis g) aufgeführten Phänomene.

Produkte zur Pflege gealterter Haut sind an sich bekannt. Sie enthalten z. B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Darüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft starke erythematöse Hautreizungen. Retinoide sind daher nur in geringen Konzentrationen einsetzbar.

Insbesondere betrifft die vorliegende Erfindung kosmetische Zubereitungen mit einem wirksamen Schutz vor schädlichen Oxidationsprozessen in der Haut.

Produkte zum Schutz vor schädlichen Oxidationsprozessen in der Haut sind an sich bekannt. Auch Produkte, welche die Sauerstoff-Versorgung der Haut steigern sollen, sind bereits im Stand der Technik beschrieben. Allerdings konnte der Stand der Technik nicht den Weg zur vorliegenden Erfindung weisen.

Die WO 02/05754 beschreibt extern applizierbare Zubereitungen, die einen Sauerstoffträger enthalten, der in einer lipoiden Emulsion molekulardispers eingearbeitet ist, sowie deren Verwendung zur externen Behandlung / Prävention von Sauerstoffmangelzuständen der Haut.

Die Patentanmeldung DE 43 25 071 offenbart ein Präparat zur Durchblutungsförderung. Zum Transport von Gasen - wie Sauerstoff oder Kohlendioxid - werden hier Fluorcarbone eingesetzt.

In der US 5,851,544 wird beschrieben, daß Hautpflegezubereitungen, welche das Hautsauerstoffniveau erhöhen, wünschenswert seien, da der Sauerstoffgehalt in der Haut niedriger sei als in anderen Körperregionen und darüber hinaus mit zunehmendem Alter abnehme. Aufgabe der US 5,851,544 ist es daher, den Sauerstoffgehalt der Haut langfristig zu steigern bzw. Sauerstoffmangelzustände in der Haut zu beheben.

All diesen Zubereitungen des Standes der Technik ist gemeinsam, dass eine gesteigerte Sauerstoffzufuhr zum dauerhaften Ausgleich eines Sauerstoffmangels explizit erreicht werden soll.

Langzeit-Sauerstofftherapien können aber auf der anderen Seite neben den oben genannten erwünschten Wirkungen bekanntermaßen auch erhebliche Nachteile haben und z. B. endogene Alterungsprozesse beschleunigen. Problematisch sind insbesondere Radikalbildung, Oxidation zellulärer Leitstrukturen und dergleichen mehr.

Es ist darüber hinaus bekannt, daß in der menschlichen und tierischen Haut unerwünschte Oxidationsprozesse auftreten können. Im Aufsatz "*Skin* Diseases *Associated with Oxidative Injury*" in *"Oxidative Stress in Dermatology",* S. 323 ff. (Marcel Decker Inc., New York, Basel, Hong Kong, Herausgeber: Jürgen Fuchs, Frankfurt, und Lester Packer, Berkeley/Californien), werden derartige oxidative Schäden der Haut und ihre näheren Ursachen aufgeführt.

Aufgabe der vorliegenden Erfindung war es daher, die Nachteile des Standes der Technik zu vermeiden und insbesondere die durch Umweltnoxen verursachten Schäden der Haut dauerhaft, nachhaltig und ohne das Risiko von Nebenwirkungen zu beheben bzw. ihnen vorzubeugen. Insbesondere sollten hautpflegende Zubereitungen und Zubereitungen zur Pflege der auf natürliche Weise gealterten Haut sowie zur Behandlung der Folgeschäden der Lichtalterung, insbesondere der unter a) bis g) aufgeführten Phänomene zur Verfügung gestellt werden. Insbesondere sollte die Wirkung der Zubereitungen physiologisch, schnell und nachhaltig sein.

Es hat sich überraschenderweise herausgestellt und darin liegt die Lösung dieser Aufgaben, daß ein Kosmetisches, nichttherapeutisches Verfahren zur Beseitigung degenerativer, alterungsassoziierter zellulärer Abläufe, wie sie in kutanen Alterungsprozess vorzufinden sind
dadurch gekennzeichnet, dass eine Emulsion, enthaltend
1 bis 90 Vol.-% nicht molekular gebundenen freien Sauerstoff, bezogen auf das Gesamtvolumen der Emülsion, und
ein Emulgatorsystem enthält, welches aus
a. mindestens einem Emulgator A, gewählt aus der Gruppe der ganz-, teil- oder nicht neutralisierten, verzweigten und/oder unverzweigten, gesättigten und/oder ungesättigten Fettsäuren mit einer Kettenlänge von 10 bis 40 Kohlenstoffatomen,
b. mindestens einem Emulgator B, gewählt aus der Gruppe der polyethoxylierten Fettsäureester mit einer Kettenlänge von 10 bis 40 Kohlenstoffatomen und mit einem Ethoxylierungsgrad von 5 bis 100 und
c. mindestens einem Coemulgator C, gewählt aus der Gruppe der gesättigten und/oder ungesättigten, verzweigten und/oder unverzweigten Fettalkohole mit einer Kettenlänge von 10 bis 40 Kohlenstoffatomen besteht,
und mindestens einmal täglich puls-artig auf die Haut aufgetragen wird, wobei die puls-artige Anwendung sich über maximal 1 Stunde erstreckt. den Nachteilen das Standes der Technik abhilft.

Die erfindungsgemäße Verwendung von Sauerstoff bzw. kosmetischer oder topischer dermatologischer Zubereitungen mit einem wirksamen Gehalt an nicht molekular gebundenem freien Sauerstoff - z. B. in Form der genannten Beispiele - führt in überraschender Weise zu einer erheblichen Verbesserung verschiedener regenerativer, aufbauender und vitalisierender Prozesse in der Haut sowie der Hautanhangsgebilde (Nägel, Haare). Insbesondere werden durch die erfindungsgemäße Verwendung die folgenden Hauteigenschaften positiv beeinflußt:
■ Abwehrfunktion,
■ Streßresistenz,
■ Nährstoffversorgung,
■ Hautfarbe und Teint,
■ Hautgeschmeidigkeit,
■ Elastizität
■ Hautstruktur,
■ Gleichgewichtszustand und Feuchtigkeitshaushalt.

Durch die Verwendung von Sauerstoff bzw. kosmetischer oder topischer dermatologischer Zubereitungen mit einem wirksamen Gehalt an Sauerstoff im Sinne der vorliegenden Erfindung wird die Haut überraschend besser gegen Alterungsprozesse, Struktur- und/oder oxidative Schäden geschützt als dies mit Zubereitungen des Standes der Technik möglich wäre. Ferner wird durch die erfindungsgemäße Verwendung die Wiederherstellung des gesunden Hautzustands im Fall einer bereits bestehenden Störung und Schädigung der normalen Hauteigenschaften begünstigt.

Unter "gestörtem Erscheinungsbild der Haut" sind im Sinne der vorliegenden Erfindung insbesondere die folgenden Phänomene zu verstehen:
■ Falten und/oder Fältchen,
■ Haut- und/oder Gewebeerschlaffung,
■ Störungen der Regeneration der Haut und der Hautanhangsgebilde,
■ Durchblutungsstörungen der Haut,
■ Altersflecken, Pigmentstörungen und sogenannter "uneven skin tone"
■ sensible Haut,
■ Juckreiz,
■ Stressempfindlichkeit der Haut,
■ entzündliche und empfindliche Hautzustände,
■ trockene Hautzustände,
■ atopisches Ekzem,
■ Psoriasis
■ Unregelmäßige bzw. zunehmende Porengröße

Es hat sich überraschend herausgestellt, daß die wesentliche Grundlage der vorliegenden Erfindung die gepulste kurzzeitige topische Applikation von molekularem Sauerstoff ist. Durch einen kurzzeitigen Puls (max. 1 Stunde) kann zwar keine längerfristige Behebung eines Sauerstoffmangels erreicht werden, so dass sich die vorliegende Erfindung grundlegend vom Stand der Technik unterscheidet. Eine kurzzeitige, puls- bzw. bolusartige Stimulation des intra- und extrazellulären Sauerstoffgehaltes hat aber für die Zellen der Haut insofern Signalfunktion, als darüber verschiedene Mechanismen angeregt werden, in deren Folge die Zellen insgesamt zellphysiologisch aktiviert werden, ohne dass dabei die Nachteile, die mit einer Langzeit-Sauerstofftherapie einhergehen, auftreten würden. Ein dauerhafter Ausgleich von Sauerstoffmangelzuständen in der Haut soll erfindungsgemäß nicht erreicht werden. Es ist aber vorteilhaft im Sinne der vorliegenden Erfindung, die pulsartige Applikation von molekularem Sauerstoff regelmäßig - z. B. 2-3 mal täglich - zu wiederholen.

Ein besonderer Vorteil der vorliegenden Erfindung ist, dass der molekulare Sauerstoff nicht an Trägerstrukturen gebunden ist. Dies hat zur Folge, daß ein schnelles An- und Abfluten des Gases durch die verschiedenen Schichten der Haut möglich ist. Lange Verweildauern und die damit einhergehenden Risiken von oxidativen und degenerativen Vorgängen, die bereits nach wenigen Stunden in einer stark sauerstoffhaltigen Atmosphäre entstehen können und dadurch dem erfinderischen Gedanken zuwider laufen würden, sind damit ausgeschlossen. Die dieser Erfindung zugrundeliegenden Daten belegen eindeutig den Vorteil einer gepulsten Applikation mit reinem molekularem Sauerstoff gegenüber einer dauerhaften Sauerstoffzufuhr oder der über Trägermoleküle.

Die erfindungsgemäße kurzzeitige Sauerstoffzufuhr, die bei Verwendung der in den Beispielen aufgeführten Applikationsformen ermöglicht wird, führt zu einer induktiven Erhöhung der regenerativen Kapazität der Haut. Der positive Effekt einer kurzzeitigen, topischen Sauerstoffbehandlung zeigt sich im wesentlichen durch die Beseitigung degenerativer, atternsassoziierter zellulärer Abläufe, wie sie im kutanen Alternsprozess vorzufinden sind. Der resultierende Effekt kann auch als Sauerstoff-Lifting ("Air-lift(ing)") bezeichnet werden.

Es ist besonders vorteilhaft im Sinne der vorliegenden Erfindung, den Sauerstoff in Form von selbstschäumenden, schaumförmigen, nachschäumenden oder schäumbaren kosmetische und dermatologische Zubereitungen auf die Haut aufzubringen.

Unter "selbstschäumend", "schaumförmig", "nachschäumend" bzw. "schäumbar" sind Zubereitungen zu verstehen, aus welchen Schäume - sei es bereits während des Herstellprozesses, sei es bei der Anwendung durch den Verbraucher oder auf andere Weise -durch Eintrag eines oder mehrerer Gase im Prinzip herstellbar sind. In derartigen Schäumen liegen die Gasbläschen (beliebig) verteilt in einer (oder mehreren) flüssigen Phase(n) vor, wobei die (aufgeschäumten) Zubereitungen makroskopisch nicht notwendigerweise das Aussehen eines Schaumes haben müssen. Erfindungsgemäße (aufgeschäumte) kosmetische oder dermatologische Zubereitungen (im folgenden der Einfachheit halber auch als Schäume bezeichnet) können z. B. makroskopisch sichtbar dispergierte Systeme aus in Flüssigkeiten dispergierten Gasen darstellen. Der Schaumcharakter kann aber beispielsweise auch erst unter einem (Licht-) Mikroskop sichtbar werden. Darüber hinaus sind erfindungsgemäße Schäume - insbesondere dann, wenn die Gasbläschen zu klein sind, um unter einem Lichtmikroskop erkannt zu werden - auch an der starken Volumenzunahme des Systems erkennbar.

Der oder die Emulgatoren A werden vorzugsweise gewählt aus der Gruppe der Fettsäuren, welche ganz oder teilweise mit üblichen Alkalien (wie z. B. Natrium- und/oder Kaliumhydroxid, Natrium- und/oder Kaliumcarbonat sowie Mono- und/oder Triethanolamin) neutralisiert sind. Besonders vorteilhaft sind beispielsweise Stearinsäure und Stearate, Isostearinsäure und Isostearate, Palmitinsäure und Palmitate sowie Myristinsäure und Myristate.

Der oder die Emulgatoren B werden vorzugsweise gewählt aus der folgenden Gruppe: PEG-9-Stearat, PEG-8-Distearat, PEG-20-Stearat, PEG-8 Stearat, PEG-8-Oleat, PEG-25-Glyceryltrioleat, PEG-40-Sorbitanlanolat, PEG-15-Glycerylricinoleat, PEG-20-Glycerylstearat, PEG-20-Glycerylisostearat, PEG-20-Glyceryloleat, PEG-20-Stearat, PEG-20-Methylglucosesesquistearat, PEG-30-Glycerylisostearat, PEG-20-Glyceryllaurat, PEG-30-Stearat, PEG-30-Glycerylstearat, PEG-40-Stearat, PEG-30-Glyceryllaurat, PEG-50-Stearat, PEG-100-Stearat, PEG-150-Laurat. Besonders vorteilhaft sind beispielsweise polyethoxylierte Stearinsäureester.

Der oder die Coemulgatoren C werden erfindungsgemäß vorzugsweise aus der folgenden Gruppe gewählt: Behenylalkohal (C₂₂H₄₅OH), Cetearylalkohol [eine Mischung aus Cetylalkohol (C₁₆H₃₃OH) und Stearylalkohol (C₁₈H₃₇OH)], Lanolinalkohole (Wollwachsalkohole, die die unverseifbare Alkoholfraktion des Wollwachses darstellen, die nach der Verseifung von Wollwachs erhalten wird). Besonders bevorzugt sind Cetyl- und Cetylstearylalkohol.

Es ist insbesondere vorteilhaft im Sinne der vorliegenden Erfindung, wenn die Zubereitungen frei von Glycerylstearat sind.

Es ist erfindungsgemäß ferner vorteilhaft, die Gewichtsverhältnisse von Emulgator A zu Emulgator B zu Coemulgator C (A : B : C) wie a : b : c zu wählen, wobei a, b und c unabhängig voneinander rationale Zahlen von 1 bis 5, bevorzugt von 1 bis 3 darstellen können. Insbesondere bevorzugt ist ein Gewichtsverhältnis von etwa 1 : 1 : 1.

Es ist vorteilhaft im Sinne der vorliegenden Erfindung, die Gesamtmenge der Emulgatoren A und B und des Coemulgators C aus dem Bereich von 2 bis 20 Gew.-%, vorteilhaft von 5 bis 15 Gew.-%, insbesondere von 7 bis 13 Gew.-%, jeweils bezogen auf das Gesamtgewicht der Formulierung, zu wählen.

Zubereitungen, welche das oben genannte Emulgatorsystem enthalten, sind insbesondere vorteilhaft im Sinne der vorliegenden Erfindung, da aus diesen bei einer einmaligen Anwendung der Sauerstoff über einen Zeitraum von weniger als einer Stunde an die Haut abgegeben wird.

Es ist ferner vorteilhaft im Sinne der vorliegenden Erfindung, wenn die kosmetischen oder dermatologischen Zubereitungen enthaltend erfindungsgemäß verwendeten Sauerstoff in Form von Gelen vorliegen. In diesem Fall enthalten die Zubereitungen im Sinne der vorliegenden Erfindung ein oder mehrere Hydrokolloide und/oder Gelbildner in einer Konzentration von 0,1 bis 8 Gew.-%, bevorzugt von 0,2 bis 6 Gew.-% und ganz besonders bevorzugt von 0,3 bis 4 Gew.-%, jeweils bezogen auf das Gesamtgewicht der Zubereitung.

"Hydrokolloid" ist die technologische Kurzbezeichnung für die an sich richtigere Bezeichnung "hydrophiles Kolloid". Hydrokolloide, auch Verdicker oder Gelbildner genannt, sind Makromoleküle, die eine weitgehend lineare Gestalt haben und über intermolekulare Wechselwirkungskräfte verfügen, die Neben- und Hauptvalenzbindungen zwischen den einzelnen Molekülen und damit die Ausbildung eines netzartigen Gebildes ermöglichen. Sie sind teilweise wasserlösliche natürliche oder synthetische Polymere, die in wässrigen Systemen Gele oder viskose Lösungen bilden. Sie erhöhen die Viskosität des Wassers, indem sie entweder Wassermoleküle binden (Hydratation) oder aber das Wasser in ihre unter sich verflochtenen Makromoleküle aufnehmen und einhüllen, wobei sie gleichzeitig die Beweglichkeit des Wassers einschränken. Solche wasserlöslichen Polymere stellen eine große Gruppe chemisch sehr unterschiedlicher natürlicher und synthetischer Polymere dar, deren gemeinsames Merkmal ihre Löslichkeit in Wasser bzw. wäßrigen Medien ist. Voraussetzung dafür ist, daß diese Polymere über eine für die Wasserlöslichkeit ausreichende Anzahl an hydrophilen Gruppen besitzen und nicht zu stark vernetzt sind. Die hydrophilen Gruppen können nichtionischer, anionischer oder kationischer Natur sein, beispielsweise wie folgt:

| | | | |
|---|---|---|---|
| -NH₂ | -COOH | -COO⁻ M⁺ | 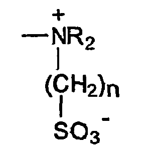 |
| -NH-R | 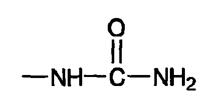 | -SO₃⁻ M⁺ | |
| -OH | | 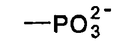 M²⁺ | |
| -SH | 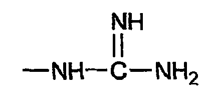 | 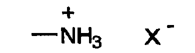 | 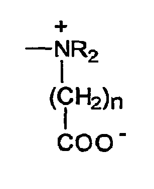 |
| -O- | | 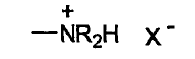 | |
| -N- | 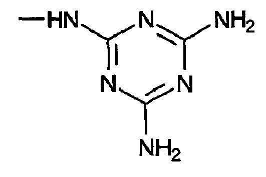 | 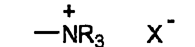 | |
| | | 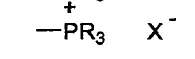 | |
| | | 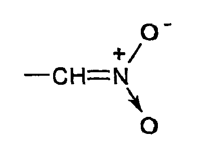 | |

Die Gruppe der kosmetisch und dermatologisch relevanten Hydrokolloide läßt sich wie folgt einteilen in:
organische, natürliche Verbindungen, wie beispielsweise Agar-Agar, Carrageen, Tragant, Gummi arabicum, Alginate, Pektine, Polyosen, Guar-Mehl, Johannisbrotbaumkernmehl, Stärke, Dextrine, Gelatine, Casein, organische, abgewandelte Naturstoffe, wie z. B. Carboxymethylcellulose und andere Celluloseether, Hydroxyethyl- und -propylcellulose und dergleichen, organische, vollsynthetische Verbindungen, wie z. B. Polyacryl- und Polymethacryl-Verbindungen, Vinylpolymere, Polycarbonsäuren, Polyether, Polyimine, Polyamide, anorganische Verbindungen, wie z. B. Polykieselsäuren, Tonmineralien wie Montmorillonite, Zeolithe, Kieselsäuren.

Als erfindungsgemäß vorteilhafte Hydrokolloide werden Agar-Agar, Carrageen, Tragant, Gummi arabicum, Alginate, Pektine, Polyosen, Guar-Mehl, Johannisbrotbaumkemmehl, Stärke, Dextrine, Gelatine, Casein, Celluloseether, Hydroxyethyl- und -propylcellulosederivate, Polyacryl- und Polymethacryl-Verbindungen, Vinylpolymere, Polycarbonsäuren, Polyether, Polyimine, Polyamide, Polykieselsäuren, Tonmineralien, Zeolithe, Kieselsäuren eingesetzt.

Erfindungsgemäß bevorzugte Hydrokolloide sind beispielsweise Methylcellulosen, als welche die Methylether der Cellulose bezeichnet werden. Sie zeichnen sich durch die folgende Strukturformel aus 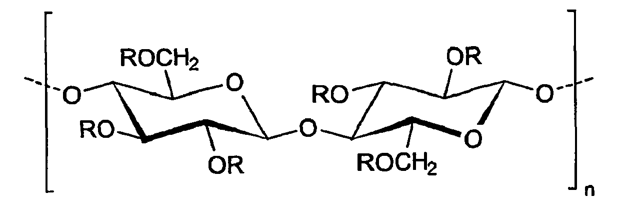 in der R ein Wasserstoff oder eine Methylgruppe darstellen kann.

Insbesondere vorteilhaft im Sinne der vorliegenden Erfindung sind die im allgemeinen ebenfalls als Methylcellulosen bezeichneten Cellulosemischether, die neben einem dominierenden Gehalt an Methyl- zusätzlich 2-Hydroxyethyl-, 2-Hydroxypropyl- oder 2-Hydroxybutyl-Gruppen enthalten. Besonders bevorzugt sind (Hydroxypropyl)methylcellulosen, beispielsweise die unter der Handelsbezeichnung Methocel E4M bei der Dow Chemical Comp. erhältlichen.

Erfindungsgemäß ferner vorteilhaft ist Natriumcarboxymethylcellulose, das Natrium-Salz des Glykolsäureethers der Cellulose, für welches R in Strukturformel I ein Wasserstoff und/oder CH2-COONa darstellen kann. Besonders bevorzugt ist die unter der Handelsbezeichnung Natrosol Plus 330 CS bei Aqualon erhältliche, auch als Cellulose Gum bezeichnete Natriumcarboxymethylcellulose.

Bevorzugt im Sinne der vorliegenden Erfindung ist ferner Xanthan (CAS-Nr. 11138-66-2), auch Xanthan Gummi genannt, welches ein anionisches Heteropolysaccharid ist, das in der Regel durch Fermentation aus Maiszucker gebildet und als Kaliumsalz isoliert wird. Es wird von Xanthomonas campestris und einigen anderen Species unter aeroben Bedingungen mit einem Molekulargewicht von 2x106 bis 24x106 produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Xanthan ist die Bezeichnung für das erste mikrobielle anionische Heteropolysaccharid. Es wird von Xanthomonas campestris und einigen anderen Species unter aeroben Bedingungen mit einem Molekulargewicht von 2-15 106 produziert. Xanthan wird aus einer Kette mit β-1,4-gebundener Glucose (Cellulose) mit Seitenketten gebildet. Die Struktur der Untergruppen besteht aus Glucose, Mannose, Glucuronsäure, Acetat und Pyruvat. Die Anzahl der Pyruvat-Einheiten bestimmt die Viskosität des Xanthans. Xanthan wird in zweitägigen Batch-Kulturen mit einer Ausbeute von 70-90 %, bezogen auf eingesetztes Kohlenhydrat, produziert. Dabei werden Ausbeuten von 25-30 g/l erreicht. Die Aufarbeitung erfolgt nach Abtöten der Kultur durch Fällung mit z. B. 2-Propanol. Xanthan wird anschließend getrocknet und gemahlen.

Vorteilhafter Gelbildner im Sinne der vorliegenden Erfindung ist ferner Carrageen, ein gelbildender und ähnlich wie Agar aufgebauter Extrakt aus nordatlantischen, zu den Florideen zählenden Rotalgen (Chondrus crispus u. Gigartina stellata).

Häufig wird die Bezeichnung Carrageen für das getrocknete Algenprodukt und Carrageenan für den Extrakt aus diesem verwendet. Das aus dem Heißwasserextrakt der Algen ausgefällte Carrageen ist ein farbloses bis sandfarbenes Pulver mit einem Molekulargewichtsbereich von 100 000-800 000 und einem Sulfat-Gehalt von ca. 25 %. Carrageen, das in warmem Wasser sehr leicht löslich ist; beim Abkühlen bildet sich ein thixotropes Gel, selbst wenn der Wassergehalt 95-98 % beträgt. Die Festigkeit des Gels wird durch die Doppelhelix-Struktur des Carrageens bewirkt . Beim Carrageenan unterscheidet man drei Hauptbestandteile: Die gelbildende κ-Fraktion besteht aus D-Galactose-4-sulfat und 3,6-Anhydro-α-D-galactose, die abwechselnd in 1,3- und 1,4-Stellung glykosidisch verbunden sind (Agar enthält demgegenüber 3,6-Anhydro-α-L-galactose). Die nicht gelierende λ-Fraktion ist aus 1,3-glykosidisch verknüpften D-Galactose-2-sulfat und 1,4-verbundenen D-Galactose-2,6-disulfat-Resten zusammengesetzt u. in kaltem Wasser leicht löslich. Das aus D-Galactose-4-sulfat in 1,3-Bindung und 3,6-Anhydro-α-D-galactose-2-sulfat in 1,4-Bindung aufgebaute τ-Carrageenan ist sowohl wasserlöslich als auch gelbildend. Weitere Carrageen-Typen werden ebenfalls mit griechischen Buchstaben bezeichnet: α, β, γ, µ, ν, ξ, π, ω, χ. Auch die Art vorhandener Kationen (K⁺, NH₄⁺, Na⁺, Mg²⁺, Ca²⁺) beeinflußt die Löslichkeit der Carrageene.

Polyacrylate sind ebenfalls vorteilhaft im sinne der vorliegenden Erfindung zu verwendende Gelatoren. Erfindungsgemäß vorteilhafte Polyacrylate sind Acrylat-Alkylacrylat-Copolymere, insbesondere solche, die aus der Gruppe der sogenannten Carbomere oder Carbopole (Carbopol® ist eigentlich eine eingetragene Marke der NOVEON Inc.) gewählt werden. Insbesondere zeichnen sich das oder die erfindungsgemäß vorteilhaften Acrylat-Alkylacrylat-Copolymere durch die folgende Struktur aus:

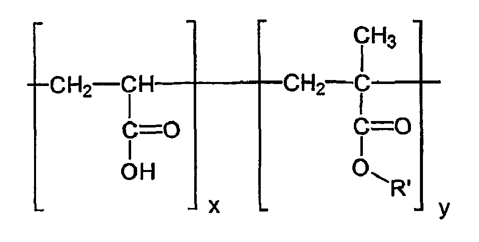

Darin stellen R' einen langkettigen Alkylrest und x und y Zahlen dar, welche den jeweiligen stöchiometrischen Anteil der jeweiligen Comonomere symbolisieren.

Erfindungsgemäß bevorzugt sind Acrylat-Copolymere und/oder Acrylat-Alkylacrylat-Copolymere, welche unter den Handelbezeichnungen Carbopol® 1382, Carbopol® 981 und Carbopol® 5984, Aqua SF-1 von der NOVEON Inc. bzw. als Aculyn® 33 von International Specialty Products Corp. erhältlich sind.

Ferner vorteilhaft sind Copolymere aus C10-30-Alkylacrylaten und einem oder mehreren Monomeren der Acrylsäure, der Methacrylsäure oder deren Ester, die kreuzvernetzt sind mit einem Allylether der Saccharose oder einem Allylether des Pentaerythrit.

Vorteilhaft sind Verbindungen, die die INCI-Bezeichnung "Acrylates/C 10-30 Alkyl Acrylate Crosspolymer" tragen. Insbesondere vorteilhaft sind die unter den Handelsbezeichnungen Pemulen TR1 und Pemulen TR2 bei der NOVEON Inc. erhältlichen.

Vorteilhaft sind ferner Verbindungen, die die INCI-Bezeichnung "acrylates/C12-24 pareth-25 acrylate copolymer" (unter der Handelsbezeichnungen Synthalen® W2000 bei der 3V Inc. erhältlich), die die INCI-Bezeichnung "acrylated/steareth-20 methacrylate copolymer" (unter der Handelsbezeichnungen Aculyn® 22 bei der International Specialty Products Corp. erhältlich), die die INCI-Bezeichnung "acrylates/steareth-20 itaconate copolymer" (unter der Handelsbezeichnungen Structure 2001® bei der National Starch erhältlich), die die INCI-Bezeichnung "acrylates/aminoacrylates/C10-30 alkyl PEG-20 itaconate copolymer" (unter der Handelsbezeichnungen Structure Plus® bei der National Starch erhältlich) und ähnliche Polymere.

Die erfindungsgemäß besonders bevorzugten Hydrokolloide sind: Acrylates Copolymer (AQUA SF-1), Acrylates/C 10-30 Alkyl Acrylate Crosspolymer (Carbopol ETD 2020), Xanthan Gum (Kelter)

Die kosmetischen oder dermatologischen Mittel gemäß der Erfindung können vorteilhaft in Form von schäumbaren Zubereitungen vorliegen, welche beispielsweise aus Aerosolbehältern entnommen und dabei aufgeschäumt werden. Erfindungsgemäß vorteilhafte Aerosolbehälter sind Sprühvorrichtungen mit einer Füllung aus den flüssigen bzw. breiartigen Stoffen, die unter dem Druck eines Treibmittels stehen (Druckgas- oder Aerosolpackungen). Derartige Behälter können mit Ventilen sehr unterschiedlicher Bauart ausgestattet sein, die die Entnahme des Inhalts als Schaum ermöglichen.

Ferner vorteilhaft können schäumbare Zubereitungen gemäß der vorliegenden Erfindung, welche das oben genannte Emulgatorsystem enthalten, aus Treibgas-freien, mechanisch zu bedienenden Pumpzerstäubern (Pumpspendern) entnommen werden. Insbesondere vorteilhaft im Sinne der vorliegenden Erfindung sind Pumpsysteme, welche ohne Druckgas, aber mit einem Filter, der spezielle Verwirbelungen bewirkt, arbeiten.

Als Druckgasbehälter kommen im Sinne der vorliegenden Erfindung vor allem zylindrische Gefäße aus Metall (Aluminium, Weißblech, Inhalt <1000 mL), geschütztem bzw. nichtsplitterndem Glas oder Kunststoff (Inhalt < 220 mL) bzw. splitterndem Glas oder Kunststoff (Inhalt < 150 mL) in Frage, bei deren Auswahl Druck- und Bruchfestigkeit, Korrosionsbeständigkeit, leichte Füllbarkeit, ggf. Sterilisierbarkeit usw., aber auch ästhetische Gesichtspunkte, Handlichkeit, Bedruckbarkeit etc. eine Rolle spielen. Der maximale zulässige Betriebsdruck von Sprüh-Dosen aus Metall bei 50 °C ist 12 bar und das maximale Füllvolumen bei dieser Temperatur ca. 90 % des Gesamtvolumens. Für Glas- und Kunststoffdosen gelten niedrigere, von der Behältergröße und dem Treibmittel (ob verflüssigtes, verdichtetes oder gelöstes Gas) abhängige Werte für den Betriebsdruck.

Besonders vorteilhaft im Sinne der vorliegenden Erfindung sind Dosen aus Weißblech, Aluminium und Glas. Aus Korrosionsschutzgründen können Metalldosen innen lackiert sein (silber- oder goldlackiert), wozu alle handelsüblichen Innenschutzlacke geeignet sind. Bevorzugt im Sinne der vorliegenden Erfindung sind Polyester-, Epoxyphenol- so wie Polyamidimidlacke. Auch Folienkaschierungen aus Polyethylen (PE), Polypropylen (PP) und/oder Polyethylenterephthalat (PET) im Innern der Dosen sind vorteilhaft, insbesondere für Dosen aus Weißblech.

Die Druckgasbehälter sind üblicherweise ein- oder zwei-, meist aber dreiteilig zylindrisch, konisch oder anders geformt. Werden Kunststoffe als Sprüh-Behältermaterial verwendet, so sollten diese Chemikalien- und Sterilisationstemperatur-beständig, gasdicht, schlagfest und gegen Innendrücke über 12 bar stabil sein. Prinzipiell für Sprüh-Behälter-Zwecke geeignet sind Polyacetale und Polyamide.

Der innere Aufbau der Sprüh-Dosen sowie die Ventilkonstruktion sind je nach Verwendungs-Zweck und der physikalischen Beschaffenheit des Inhalts - z. B. ob als Zwei- oder als Dreiphasensystem - sehr variantenreich und können vom Fachmann durch einfaches Ausprobieren ohnen erfinderisches Zutun ermittelt werden. Für geeignete Ausführungsformen sei auf das "Aerosol Technologie Handbuch der Aersosol-Verpackung" hingewiesen (*Wolfgang Tauscher, Melcher Verlag GmbH HeidelberglMünchen, 1996*).

Erfindungsgemäß vorteilhafte Ventile können mit oder ohne Steigrohr ausgebildet sein. Die Einzelteile, aus welchen erfindungsgemäße Ventile üblicherweise aufgebaut sind, bestehen vorzugsweise aus den folgenden Materialien:
- Teller:: Weißblech: blank, gold- bzw. klarlackiert, folienkaschiert (PE, PP oder PET) Aluminium: blank, silber- oder goldlackiert, verschiedene Lackvarianten, Stoner- Mudge-Ausführung
- Dichtung:: natürliche bzw. synthetische Elastomere bzw. thermoplastische (Sleeve- Gaskets, folienkaschiert aus PE oder PP) Innen- und Aussendichtungen, z. B. aus Perbunan, Buna, Neopren, Butyl, CLB, LDPE, Viton, EPDM, Chlorbutyl, Brombutyl und/oder diversen Compounds
- Kegel:: PA, POM, Messing sowie diversen Sondermaterialen, Standardbohrungen (z. B.: 0,25 bis 0,70 mm oder 2 x 0,45 bis 2 x 1,00 mm), verschiedene Schaftdurchmesser
- Feder:: Metall, besonders bevorzugt V2A, rostfreier Stahl; Kunststoff und auch Elastomer
- Gehäuse:: Standard und Impact VPH-Bohrungen, RPT-Bohrungen oder geschlitzt für Überkopf-Anwendungen Materialien: z. B. Polyacetal, PA, PE, POM und dergleichen mehr
- Steigrohr:: Kunststoff (Polymer Resin), z.B. PE, PP, PA oder Polycarbonat

Vorteilhafte Sprühköpfe im Sinne der vorliegenden Erfindung sind beispielsweise Schaumköpfe für die aufrechte Anwendung (Dose senkrecht halten) oder Schaumköpfe für die Überkopf-Anwendung mit einem oder mehreren Kanälen.

Vorteilhaft im Sinne der vorliegenden Erfindung wird der Volumenanteil an Treibgas aus dem Bereich von 0,1 bis 30 Vol.-%, bezogen auf das Gesamtvolumen aus Füllgut und Treibgas gewählt (entsprechend einem Volumenanteil von 70 bis 99,9 Vol.-% Füllgut).

Besonders bevorzugtes Treibgas im Sinne der vorliegenden Erfindung ist Sauerstoff, obwohl Luft bzw. andere Sauerstoff enthaltende Gasmischungen prinzipiell auch geeignet ist.

Es ist vorteilhaft im Sinne der vorliegenden Erfindung, die Gesamtmenge an Sauerstoff in den aufgeschäumten Zubereitungen im Sinne der vorliegenden Erfindung aus dem Bereich von 0,5 bis 90 Vol.-%, vorteilhaft von 5 bis 50 Vol.-%, insbesondere von 10 bis 40 Vol.-%, jeweils bezogen auf das Gesamtvolumen der Formulierung, zu wählen.

Die kosmetischen oder dermatologischen Formulierungen im Sinne der vorliegenden Erfindung können wie üblich zusammengesetzt sein und dem kosmetischen oder dermatologischen Lichtschutz, ferner zur Behandlung, Pflege und Reinigung der Haut, der Lippen, sowie von Hautanhangsgebilden (Nägel und/oder Haare) und als Schminkprodukt in der dekorativen Kosmetik dienen.

Ebenso wie Emulsionen von flüssiger und fester Konsistenz als kosmetische Reinigungslotionen bzw. Reinigungscremes Verwendung finden, können auch die Zubereitungen im Sinne der vorliegenden Erfindung "Reinigungsschäume" darstellen, welche beispielsweise zum Entfernen von Schminken und/oder Make-up oder als milder Wasch- und Duschschaum - ggf. auch für unreine Haut - verwendet werden können. Derartige Reinigungsschäume können vorteilhaft ferner als sogenannte "rinse off" Präparate angewendet werden, welche nach der Anwendung von der Haut abgespült werden

Die erfindungsgemäßen kosmetischen und/oder dermatologischen Zubereitungen können auch vorteilhaft in Form eines Schaums zur Pflege des Haars bzw. der Kopfhaut vorliegen, insbesondere eines Schaums zum Einlegen der Haare, eines Schaums, der beim Fönen der Haare verwendet wird, eines Frisier- und Behandlungsschaums.

Entsprechend ihrem Aufbau können kosmetische oder topische dermatologische Zusammensetzungen im Sinne der vorliegenden Erfindung, beispielsweise verwendet werden als Hautschutzcrème, Reinigungsmilch, Tages- oder Nachtcrème usw. Es ist gegebenenfalls möglich und vorteilhaft, die erfindungsgemäßen Zusammensetzungen als Grundlage für pharmazeutische Formulierungen zu verwenden.

Zur Anwendung werden die kosmetischen und dermatologischen Zubereitungen in der für Kosmetika üblichen Weise auf die Haut und/oder die Haare in ausreichender Menge aufgebracht.

Die kosmetischen und dermatologischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Steigern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Vorteilhafte Konservierungsmittel im Sinne der vorliegenden Erfindung sind beispielsweise Formaldehydabspalter (wie z. B. DMDM Hydantoin, welches beispielsweise unter der Handelsbezeichnung Glydant^{™} von der Fa. Lonza erhältlich ist), Iodopropylbutylcarbamate (z. B. die unter den Handelsbezeichnungen Glycacil-L, Glycacil-S von der Fa. Lonza erhältlichen und/oder Dekaben LMB von Jan Dekker), Parabene (d. h. p-Hydroxybenzoesäurealkylester, wie Methyl-, Ethyl-, Propyl- und/oder Butylparaben), Phenoxyethanol, Ethanol, Benzoesäure und dergleichen mehr. Üblicherweise umfaßt das Konservierungssystem erfindungsgemäß ferner vorteilhaft auch Konservierungshelfer, wie beispielsweise Octoxyglycerin, Glycine Soja etc.

Vorteilhafte Komplexbildner im Sinne der vorliegenden Erfindung sind beispielsweise EDTA, [S,S]-Ethylendiamindisuccinat (EDDS), welches beispielsweise unter der Handelsbezeichnung Octaquest von der Fa. Octel erhältlich ist, Pentanatrium-Ethylendiamintetramethylenphosphonat, welches z. B. unter dem Handelsnamen Dequest 2046 von der Fa. Monsanto erhältlich ist und/oder Iminodibersteinsäure, welche u. a. von der Fa. Bayer AG unter den Handelsnamen Iminodisuccinat VP OC 370 (ca. 30% ige Lösung) und Baypure CX 100 fest erhältlich ist.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Bevorzugte Antioxidantien sind ferner Vitamin E und dessen Derivate sowie Vitamin A und dessen Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische oder dermatologische Wirkstoffe enthalten, wobei bevorzugte Wirkstoffe Antioxidantien sind, welche die Haut vor oxidativer Beanspruchung schützen können.

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin,Kreatinin, Taurin und/oder β-Alanin sowie 8-Hexadecen-1,16-dicarbonsäure (Dioic acid, CAS-Nummer 20701-68-2; vorläufige INCI-Bezeichnung Octadecendioic acid).

Erfindungsgemäße Rezepturen, welche z. B. bekannte Antifaltenwirkstoffe wie Flavonglycoside (insbesondere α-Glucosylrutin), Coenzym Q10, Vitamin E und/oder Derivate und dergleichen enthalten, eignen sich insbesondere vorteilhaft zur Prophylaxe und Behandlung kosmetischer oder dermatologischer Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Couperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissig keit) und dergleichen). Weiterhin vorteilhaft eignen sie sich gegen das Erscheinungsbild der trockenen bzw. rauhen Haut.

Es ist besonders vorteilhaft im Sinne der vorliegenden Erfindung, wenn die weiteren Wirkstoffe in verkapselter Form vorliegen, so daß sie pysikalisch getrennt von den Formulierungsbestandteilen (oder weiteren, nicht kompatiblen Wirkstoffen) und/oder dem potentiell oxidierend wirkenden Sauerstoff vorliegen. In diesem Zusammenhang sind - je nach Art der eingesetzten Wirkstoffes - permanente Verkapselungen, d. h. Kapseln, aus denen die Wirkstoffe nicht in die kosmetische Zubereitung oder die Haut abgegeben werden (vorteilhaft z. B. für UV-Filtersubstanzen) oder nicht permanente Verkapselungen denkbar.

Vorteilhafte Verkapselungen bestehen z. B. Kunststoffen. Es ist ferner vorteilhaft, die weiteren Wirkstoffe in Kollagenmatrices und anderen üblichen Verkapselungsmaterialien zu verkapseln, z. B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt. Besonders vorteilhafte Verkapselungsformen im Sinne der vorliegenden Erfindung sind ferner Cyclodextrinkomplexe der weiteren Wirkstoffe.

Vorteilhaft sind auch z. B. Verkapselungen, die nach der Sol-Gel-Mikrotechnologie erhältlich sind. Hierbei werden die Wirkstoffe in einer inerten Silica Membran eingeschlossen, letztendlich also in Glasperlen verkapselt. Vorteilhaft im Sinne der vorliegenden Erfindung können verkapselte Wirkstoffe auch in Form von wäßrigen Dispersionen eingesetzt werden.

Verkapselte Wirkstoffe eignen sich insbesondere zur Herstellung von besonders hautverträglichen (Sensitiv-) Produkten. Darüber hinaus ist es selbstverständlich vorteilhaft, potentiell hautreizende Wirkstoffe in verkapselter Form einzusetzen.

Ferner ist es auch vorteilhaft im Sinne der vorliegenden Erfindung, als "microbeads" bezeichnete Wirkstoffkapseln einzusetzen. Vorteilhafte "microbeads" sind z. B. die im folgenden aufgelisteten:

| **Handelsname** | **Hersteller** | **Zusammensetzung (INCI)** |
|---|---|---|
| Unispheres UEL-611 | Induchem | Lactose + Cellulose + Hydroxypropyl Methylcellulose + Cl 77707 + Toco-pherolacetate |
| Unipheres RP-572 | Induchem | Lactose + Cellulose + Hydroxpropyl Methylcellulose + Panthenyl Triacetate + Cl 7360 |
| Unipheres UT-513 | Induchem | Lactose + Cellulose + Hydroxypropyl Methylcellulose + Cl 77707 + Tocopherol |
| Macrobeads | Wiblosan | Cetearyl Alkohol + Acrylates Copolymer + Paraffinum Liquidum + Microcristalline Cellulose + Bisabolo I+ Tocopherol Acetate + Cl 74260 |

Die Wasserphase der Zubereitungen gemäß der vorliegenden Erfindung kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Butylenglykol, Ethylenglykol, Ethylenglykolmonoethyl- oder -monobutylether, Propylenglykolmonomethyl, - monoethyl- oder -monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole [von der Fa. Bf. Goodrich], beispielsweise Carbopole der Typen 980, 981, 1382, 2984, 5984, ETD 2020, ETD 2050, Ultrez 10, jeweils einzeln oder in Kombination.

Die Zubereitungen gemäß der vorliegenden Erfindung können ferner vorteilhaft auch Selbstbräunungssubstanzen enthalten, wie beispielsweise Dihydroxyaceton und/oder Melaninderivate in Konzentrationen von 1 Gew.-% bis zu 8 Gew.-%, bezogen auf das Gesamtgewicht der Zubereitung.

Ferner vorteilhaft können die Zubereitungen gemäß der vorliegenden Erfindung auch Repellentien zum Schutz vor Mücken, Zecken und Spinnen und dergleichen enthalten. Vorteilhaft sind z. B. N,N-Diethyl-3-methylbenzamid (Handelsbezeichnung: Meta-delphene, "DEET"), Dimethylphtalat (Handelsbezeichnung: Palatinol M, DMP) sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Als Moisturizer werden Stoffe oder Stoffgemische bezeichnet, welche kosmetischen oder dermatologischen Zubereitungen die Eigenschaft verleihen, nach dem Auftragen bzw. Verteilen auf der Hauptoberfläche die Feuchtigkeitsabgabe der Hornschicht (auch transepidermal water loss (TEWL) genannt) zu reduzieren und/oder die Hydratation der Hornschicht positiv zu beeinflussen.

Vorteilhafte Moisturizer im Sinne der vorliegenden Erfindung sind beispielsweise Glycerin, Milchsäure und/oder Lactate, insbesondere Natriumlactat, Butylenglykol, Propylenglykol, Biosaccaride Gum-1, Glycine Soja, Ethylhexyloxyglycerin, Pyrrolidoncarbonsäure und Harnstoff. Ferner ist es insbesondere von Vorteil, polymere Moisturizer aus der Gruppe der wasserlöslichen und/oder in Wasser quellbaren und/oder mit Hilfe von Wasser gelierbaren Polysaccharide zu verwenden. Insbesondere vorteilhaft sind beispielsweise Hyaluronsäure, Chitosan und/oder ein fucosereiches Polysaccharid, welches in den Chemical Abstracts unter der Registraturnummer 178463-23-5 abgelegt und z. B. unter der Bezeichnung Fucogel®1000 von der Gesellschaft SOLABIA S.A. erhältlich ist. Moisturizer können vorteilhaft auch als Antifaltenwirkstoffe zur Prophylaxe und Behandlung kosmetischer oder dermatologischer Hautveränderungen, wie sie z. B. bei der Hautalterung auftreten, verwendet werden.

Die erfindungsgemäßen kosmetischen oder dermatologischen Zubereitungen können ferner vorteilhaft, wenngleich nicht zwingend, Füllstoffe enthalten, welche z. B. die sensorischen und kosmetischen Eigenschaften der Formulierungen weiter verbessern und beispielsweise ein samtiges oder seidiges Hautgefühl hervorrufen oder verstärken. Vorteilhafte Füllstoffe im Sinne der vorliegenden Erfindung sind Stärke und Stärkederivate (wie z. B. Tapiocastärke, Distärkephosphat, Aluminium- bzw. Natrium-Stärke Octenylsuccinat und dergleichen), Pigmente, die weder hauptsächlich UV-Filter- noch färbende Wirkung haben (wie z. B. Bornitrid etc.) und/oder Aerosile^{®} (CAS-Nr. 7631-86-9).

Die Ölphase der erfindungsgemäßen Formulierungen wird vorteilhaft gewählt aus der Gruppe der polaren Öle, beispielsweise aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, wie z. B. Cocoglycerid, Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl und dergleichen mehr.

Erfindungsgemäß vorteilhaft sind ferner z. B. natürliche Wachse tierischen und pflanzlichen Ursprungs, wie beispielsweise Bienenwachs und andere Insektenwachse sowie Beerenwachs, Sheabutter und/oder Lanolin (Wollwachs).

Weitere vorteilhafte polare Ölkomponenten können im Sinne der vorliegenden Erfindung ferner gewählt werden aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen sowie aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Octylpalmitat, Octylcocoat, Octylisostearat, Octyldodeceylmyristat, Octyldodekanol, Cetearylisononanoat, Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Stearylheptanoat, Oleyloleat, Oleylerucat, Erucyloleat, Erucylerucat, Tridecylstearat, Tridecyltrimellitat, sowie synthetische, halbsynthetische und natürliche Gemische solcher Ester, wie z. B. Jojobaöl.

Ferner kann die Ölphase vorteilhaft gewählt werden aus der Gruppe der Dialkylether und Dialkylcarbonate, vorteilhaft sind z. B. Dicaprylylether (*Cetiol OE*) und/oder Dicaprylylcarbonat, beispielsweise das unter der Handelsbezeichnung *Cetiol CC* bei der Fa. Cognis erhältliche.

Es ist ferner bevorzugt, das oder die Ölkomponenten aus der Gruppe Isoeikosan, Neopentylglykoldiheptanoat, Propylenglykoldicaprylatldicaprat, Caprylic/Capric/Diglycerylsuccinat, Butylenglykol Dicaprylat/Dicaprat, C₁₂₋₁₃-Alkyllactat, Di-C₁₂₋₁₃-Alkyltartrat, Triisostearin, Dipentaerythrityl Hexacaprylat/Hexacaprat, Propylenglykolmonoisostearat, Tricaprylin, Dimethylisosorbid. Es ist insbesondere vorteilhaft, wenn die Ölphase der erfindungsgemäßen Formulierungen einen Gehalt an C₁₂₋₁₅-Alkylbenzoat aufweist oder vollständig aus diesem besteht.

Vorteilhafte Ölkomponenten sind ferner z. B. Butyloctylsalicylat (beispielsweise das unter der Handelsbezeichnung *Hallbrite BHB* bei der Fa. CP Hall erhältliche), Hexadecylbenzoat und Butyloctylbenzoat und Gemische davon (*Hallstar AB*) und/oder Diethylhexylnaphthalat *(Hallbrite TQ oder Corapan TQ von H&R).*

Auch beliebige Abmischungen solcher Öl- und Wachskomponenten sind vorteilhaft im Sinne der vorliegenden Erfindung einzusetzen.

Ferner kann die Ölphase ebenfalls vorteilhaft auch unpolare Öle enthalten, beispielsweise solche, welche gewählt werden aus der Gruppe der verzweigten und unverzweigten Kohlenwasserstoffe und -wachse, insbesondere Mineralöl, Vaseline (Petrolatum), Paraffinöl, Squalan und Squalen, Polyolefine, hydrogenierte Polyisobutene und Isohexadecan. Unter den Polyolefinen sind Polydecene die bevorzugten Substanzen.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Silikonöle sind hochmolekulare synthetische polymere Verbindungen, in denen SiliciumAtome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind. Systematisch werden die Silikonöle als Polyorganosiloxane bezeichnet. Die methylsubstituierten Polyorganosiloxane, welche die mengenmäßig bedeutendsten Verbindungen dieser Gruppe darstellen und sich durch die folgende Strukturformel auszeichnen 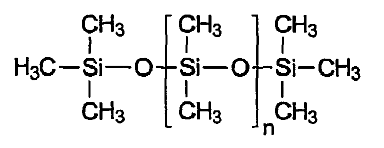 werden auch als Polydimethylsiloxan bzw. Dimethicon (INCI) bezeichnet. Dimethicone gibt es in verschiedenen Kettenlängen bzw. mit verschiedenen Molekulargewichten.

Besonders vorteilhafte Polyorganosiloxane im Sinne der vorliegenden Erfindung sind beispielsweise Dimethylpolysiloxane [Poly(dimethylsiloxan)], welche beispielsweise unter den Handelsbezeichnungen Abil 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silikone (Octamethylcyclotetrasiloxan bzw. Decamethylcyclopentasiloxan), welche nach INCI auch als Cyclomethicone bezeichnet werden, aminomodifizierte Silikone (INCI: Amodimethicone) und Silikonwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind. Aber auch andere Silikonöle sind vorteilhaft im Sinne der vorliegenden Erfindung zu verwenden, beispielsweise Cetyldimethicon, Hexamethylcyclotrisiloxan, Polydimethylsiloxan, Poly(methylphenylsiloxan).

Die Zubereitungen gemäß der vorliegenden Erfindung können ferner vorteilhaft eine oder mehrere Substanzen aus der folgenden Gruppe der Siloxanelastomere enthalten, beispielsweise um die Wasserfestigkeit und/oder den Lichtschutzfaktor der Produkte zu steigern:
(a) Siloxanelastomere, welche die Einheiten R₂SiO und RSiO_{1,5} und/oder R₃SiO_{0,5} und/oder SiO₂ enthalten,
   wobei die einzelnen Reste R jeweils unabhängig voneinander Wasserstoff, C₁₋₂₄-Alkyl (wie beispielsweise Methyl, Ethyl, Propyl) oder Aryl (wie beispielsweise Phenyl oder Tolyl), Alkenyl (wie beispielsweise Vinyl) bedeuten und das Gewichtsverhältnis der Einheiten R₂SiO zu RSiO_{1,5} aus dem Bereich von 1 : 1 bis 30 : 1 gewählt wird;
(b) Siloxanelastomere, welche in Silikonöl unlöslich und quellfähig sind, die durch die Additionsreaktion eines Organopolysiloxans (1), das siliciumbebundenen Wasserstoff enthält, mit einem Organopolysiloxan (2), das ungesättigte aliphatische Gruppen enthält, erhältlich sind,
   wobei die verwendeten Mengenateile so gewählt werden, daß die Menge des Wasserstoffes des Organopolysiloxans (1) oder der ungesättigten aliphatischen Gruppen des Organopolysiloxans (2)
   - im Bereich von 1 bis 20 mol-% liegt, wenn das Organopolysiloxan nicht cyclisch ist und
   - im Bereich von 1 bis 50 mol-% liegt, wenn das Organopolysiloxan cyclisch ist.

Vorteilhaft im Sinne der vorliegenden Erfindung liegen das oder die Siloxanelastomere in Form sphärischer Puder oder in Form von Gelen vor.

Erfindungsgemäß vorteilhafte in Form sphärischer Puder vorliegende Siloxanelastomere sind die mit der INCI-Bezeichnung Dimethicone / Vinyl Dimethicone Crosspolymer, beispielsweise das von DOW CORNING unter der Handelsbezeichnungen DOW CORNING 9506 Powder erhältliche.

Besonders bevorzugt ist es, wenn das Siloxanelastomer in Kombination mit Ölen aus Kohlenwasserstoffen tierischer und/oder pflanzlicher Herkunft, synthetischen Ölen, synthetischen Estern, synthetischen Ethern oder deren Gemischen verwendet wird.

Ganz besonders bevorzugt ist es, wenn das Siloxanelastomer in Kombination mit unverzweigten bei Raumtemperatur flüssigen oder pastösen Silikonölen oder cyclischen Sili konölen oder deren Gemischen verwendet wird. Insbesondere vorteilhaft sind Organopolysiloxanelastomere mit der INCI-Bezeichnung Dimethicone / Polysilicone-11, ganz besonders die von der Grant Industries Inc. erhältlichen Gransil-Typen GCM, GCM-5, DMG-6, CSE Gel, PM-Gel, LTX, ININ Gel, AM-18 Gel und/oder DMCM-5.

Ganz außergewöhnlich bevorzugt ist es, wenn das Siloxanelastomer in Form eines Gels aus Siloxanelastomer und einer Lipidphase verwendet wird, wobei der Gehalt des Siloxanelastomers in dem Gel 1 bis 80 Gew.-%, bevorzugt 0,1 bis 60 Gew.-% beträgt, jeweils bezogen auf das Gesamtgewicht des Gels.

Es ist vorteilhaft im Sinne der vorliegenden Erfindung, die Gesamtmenge der Siloxanelastomere (Aktivgehalt) aus dem Bereich von 0,01 bis 10 Gew.-%, vorteilhaft von 0,1 bis 5 Gew.-%, jeweils bezogen auf das Gesamtgewicht der Formulierung, zu wählen.

Die erfindungsgemäßen kosmetischen und dermatologischen Zubereitungen können Farbstoffe und/oder Farbpigmente enthalten, insbesondere wenn sie in Form von dekorativen Kosmetika vorliegen. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. Fe₂O₃, Fe₃O₄, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus dem *Rowe Colour Index, 3. Auflage, Society of Dyers and Colourists, Bradford, England, 1971* zu wählen.

Sofern die erfindungsgemäßen Formulierungen in Form von Produkten vorliegen, welche im Gesicht angewendet werden, ist es günstig, als Farbstoff eine oder mehrere Substanzen aus der folgenden Gruppe zu wählen: 2,4-Dihydroxyazobenzol, 1-(2'-Chlor-4'-nitro-1'-phenylazo)-2-hydroxynaphthalin, Ceresrot, 2-(Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 4-(4-Sulfo-1-phenylazor-1-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN. 77 491 (rot) und 77 499 (schwarz)), Eisenoxidhydrat (CIN: 77 492), Mangan ammoniumdiphosphat und Titandioxid.

Ferner vorteilhaft sind öllösliche Naturfarbstoffe, wie z. B. Paprikaextrakte, -Carotin oder Cochenille.

Vorteilhaft im Sinne der vorliegenden Erfindung sind ferner Formulierungen mit einem Gehalt an Perlglanzpigmenten. Bevorzugt sind insbesondere die im folgenden aufgelisteten Arten von Perlglanzpigmenten:
1. Natürliche Perlglanzpigmente, wie z. B.
   ■ "Fischsilber" (Guanin/Hypoxanthin-Mischkristalle aus Fischschuppen) und
   ■ "Perlmutt" (vermahlene Muschelschalen)
2. Monokristalline Perlglanzpigmente wie z. B. Bismuthoxychlorid (BiOCI)
3. Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteihaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| Gruppe | Belegung / Schichtdicke | Farbe |
|---|---|---|
| Silberweiße Perlglanzpigmente | TiO₂: 40 - 60 nm | silber |
| Interferenzpigmente | TiO₂: 60 - 80 nm | gelb |
| | TiO₂: 80 -100 nm | rot |
| | TiO₂: 100 - 140 nm | blau |
| | TiO₂: 120 - 160 nm | grün |
| Farbglanzpigmente | Fe₂O₃ | bronze |
| | Fe₂O₃ | kupfer |
| | Fe₂O₃ | rot |
| | Fe₂O₃ | rotviolett |
| | Fe₂O₃ | rotgrün |
| | Fe₂O₃ | schwarz |
| Kombinationspigmente | TiO₂ /Fe₂O₃ | Goldtöne |
| | TiO₂ / Cr₂O₃ | grün |
| | TiO₂ / Berliner Blau | tiefblau |
| | TiO₂ / Carmin | rot |

Besonders bevorzugt sind z. B. die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit TiO₂ und Fe₂O₃ beschichtete SiO₂-Partikel ("Ronaspheren"), die von der Firma Merck vertrieben werden und sich besonders für die optische Reduktion feiner Fältchen eignen.

Es kann darüber hinaus von Vorteil sein, gänzlich auf ein Substrat wie Glimmer zu verzichten. Besonders bevorzugt sind Eisenperlglanzpigmente, welche ohne die Verwendung von Glimmer hergestellt werden. Solche Pigmente sind z. B. unter dem Handelsnamen Sicopearl Kupfer 1000 bei der Firma BASF erhältlich.

Besonders vorteilhaft sind ferner auch Effektpigmente, welche unter der Handelsbezeichnung Metasomes Standard / Glitter in verschiedenen Farben (yello, red, green, blue) von der Firma Flora Tech erhältlich sind. Die Glitterpartikel liegen hierbei in Gemischen mit verschiedenen Hilfs- und Farbstoffen (wie beispielsweise den Farbstoffen mit den Colour Index (Cl) Nummern 19140, 77007, 77289, 77491) vor.

Die Farbstoffe und Pigmente können sowohl einzeln als auch im Gemisch vorliegen sowie gegenseitig miteinander beschichtet sein, wobei durch unterschiedliche Beschichtungsdikken im allgemeinen verschiedene Farbeffekte hervorgerufen werden. Die Gesamtmenge der Farbstoffe und farbgebenden Pigmente wird vorteilhaft aus dem Bereich von z. B. 0,1 Gew.-% bis 30 Gew.-%, vorzugsweise von 0,5 bis 15 Gew.-%, insbesondere von 1,0 bis 10 Gew.-% gewählt, jeweils bezogen auf das Gesamtgewicht der Zubereitungen.

Es ist auch vorteilhaft im Sinne der vorliegenden Erfindung, kosmetische und dermatologische Zubereitungen zu erstellen, deren hauptsächlicher Zweck nicht der Schutz vor Sonnenlicht ist, die aber dennoch einen Gehalt an weiteren UV-Schutzsubstanzen enthalten. So werden z. B. in Tagescremes oder Makeup-Produkten gewöhnlich UV-A- bzw. UV-B-Filtersubstanzen eingearbeitet. Auch stellen UV-Schutzsubstanzen, ebenso wie Antioxidantien und, gewünschtenfalls, Konservierungsstoffe, einen wirksamen Schutz der Zubereitungen selbst gegen Verderb dar. Günstig sind ferner kosmetische und dermatologische Zubereitungen, die in der Form eines Sonnenschutzmittels vorliegen.

Dementsprechend enthalten die Zubereitungen im Sinne der vorliegenden Erfindung vorzugsweise mindestens eine weitere UV-A-, UV-B- und/oder Breitbandfiltersubstanz. Die Formulierungen können, obgleich nicht notwendig, gegebenenfalls auch ein oder mehrere organische und/oder anorganische Pigmente als UV-Filtersubstanzen enthalten, welche in der Wasser und/oder der Ölphase vorliegen können.

Die Zubereitungen gemäß der vorliegenden Erfindung können ferner vorteilhaft auch in Form von sogenannten ölfreien kosmetischen oder dermatologischen Emulsionen vorliegen, welche eine Wasserphase und mindestens eine bei Raumtemperatur flüssige UV-Filtersubstanz als weitere Phase enthalten und welche insbesondere vorteilhaft auch frei von weiteren Ölkomponenten sein können.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-cyano-3,3-diphenylacrylat (INCI: Octocrylene), 2-Ethylhexyl-2-hydroxybenzoat (2-Ethylhexylsalicylat, Octylsalicylat, INCI: Ethylhexyl Salicylate) und Ester der Zimtsäure, vorzugsweise 4-Methoxyzimtsäure(2-ethylhexyl)ester (2-Ethylhexyl-4-methoxycinnamat, INCI: Ethylhexyl Methoxycinnamate) und 4-Methoxyzimtsäureisopentylester (Isopentyl-4-methoxycinnamat, INCI: Isoamyl p-Methoxycinnamate), 3-(4-(2,2-bis Ethoxycarbonylvinyl)-phenoxy)propenyl)-methoxysiloxan/Dimethylsiloxan - Copolymer welches beispielsweise unter der Handelsbezeichnung Parsol® SLX bei Hoffmann La Roche erhältlich ist.

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden sowie das Sulfat des Bariums (BaSO₄).

Die Pigmente können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Die Pigmente können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, daß die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß geeignete Zinkoxidpartikel und Vordispersionen von Zinkoxidpartikeln sind unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| Z- Cote | / | BASF |
| ZnO NDM | 5% Dimethicone | H&R |
| MZ- 303S | 3% Methicone | Tayca Corporation |
| MZ- 505S | 5% Methicone | Tayca Corporation |

Geeignete Titandioxidpartikel und Vordispersionen von Titandioxidpartikeln sind unter folgenden Handelsbezeichnungen bei den aufgeführten Firmen erhältlich:

| **Handelsname** | **Coating** | **Hersteller** |
|---|---|---|
| MT-100TV | Aluminiumhydroxid / Stearinsäure | Tayca Corporation |
| MT-100Z | Aluminiumhydroxid / Stearinsäure | Tayca Corporation |
| Eusolex T-2000 | Alumina / Simethicone | Merck KgaA |
| Titandioxid T805 (Uvinul TiO₂) | Octyltrimethylsilan | Degussa |
| Tioveil AQ 10PG | Alumina / Silica | Solaveil / Uniquema |
| Eusolex T-aqua | Wasser/Alumina/Natriummeta phosphat | Merck |

Weitere vorteilhafte Pigmente sind Latexpartikel. Erfindungsgemäß vorteilhafte Latexpartikel sind die in den folgenden Schriften beschriebenen: US 5,663,213 bzw. EP 0 761 201. Besonders vorteilhafte Latexpartikel sind solche, welche aus Wasser und Styrol/Acrylat-Copolymeren gebildet werden und z. B. unter der Handelsbezeichnung "Alliance SunSphere" bei der Fa. Rohm & Haas erhältlich sind.

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol^{®} 1789 und von Merck unter der Handelsbezeichnung Eusolex® 9020 verkauft wird.

Vorteilhafte weitere UV-Filtersubstanzen im Sinne der vorliegenden Erfindung sind sulfonierte, wasserlösliche UV-Filter, wie z. B.:
- Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Disodium Phenyl Dibenzimidazol Tetrasulfonat (CAS-Nr.: 180898-37-7), welches beispielsweise unter der Handelsbezeichnung Neo Heliopan AP bei Haarmann & Reimer erhältlich ist;
- Salze der 2-Phenylbenzimidazol-5-sulfonsäure, wie ihr Natrium-, Kalium-oder ihr Triethanolammonium-Salz sowie die Sulfonsäure selbst mit der INCI Bezeichnung Phenylbenzimidazole Sulfonsäure (CAS.-Nr. 27503-81-7), welches beispielsweise unter der Handelsbezeichnung Eusolex 232 bei Merck oder unter Neo Heliopan Hydro bei Haarmann & Reimer erhältlich ist;
- 1,4-di(2-oxo-10-Sulfo-3-bornylidenmethyl)-Benzol (auch: 3,3'-(1,4-Phenylendimethylene)-bis-(7,7-dimethyl-2-oxo-bicydo-[2.2.1]hept-1-ylmethan Sulfonsäure) und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird. Benzol-1,4-di(2-oxo-3-bornylidenmethyl-10-sulfonsäure) hat die INCI-Bezeichnung Terephtalidene Dicampher Sulfonsäure (CAS.-Nr.: 90457-82-2) und ist beispielsweise unter dem Handelsnamen Mexoryl SX von der Fa. Chimex erhältlich;
- Sulfonsäure-Derivate des 3-Benzylidencamphers, wie z. B. 4 -(2-Oxo-3-bornylidenmethyl)benzolsulfonsäure, 2-Methyl-5-(2-oxo-3-bornylidenmethyl)sulfonsäure und deren Salze.

Vorteilhafte UV-Filtersubstanzen im Sinne der vorliegenden Erfindung sind ferner Benzoxazol-Derivate, welche sich durch die folgende Strukturformel auszeichnen, 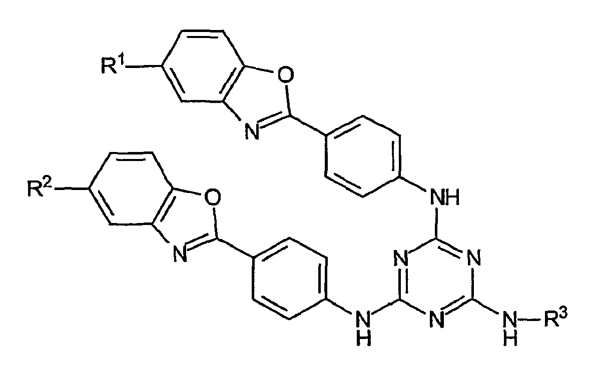 worin R¹, R² und R³ unabhängig voneinander gewählt werden aus der Gruppe der verzweigten oder unverzweigten, gesättigten oder ungesättigten Alkylreste mit 1 bis 10 Kohlenstoffatomen. Es ist erfindungsgemäß besonders vorteilhaft, die Reste R¹ und R² gleich zu wählen, insbesondere aus der Gruppe der verzweigten Alkylreste mit 3 bis 5 Kohlenstoffatomen. Es ist ferner besonders vorteilhaft im Sinne der vorliegenden Erfindung, wenn R³ einen unverzweigten oder verzweigten Alkylrest mit 8 Kohlenstoffatomen, insbesondere den 2-Ethylhexylrest darstellt.

Erfindungsgemäß besonders bevorzugtes Benzoxazol-Derivat ist das 2,4-bis-[5-1(dimethylpropyl)benzoxazol-2-yl-(4-phenyl)-imino]-6-(2-ethylhexyl)-imino-1,3,5-triazin mit der CAS Nr. 288254-16-0, welches sich durch die Strukturformel 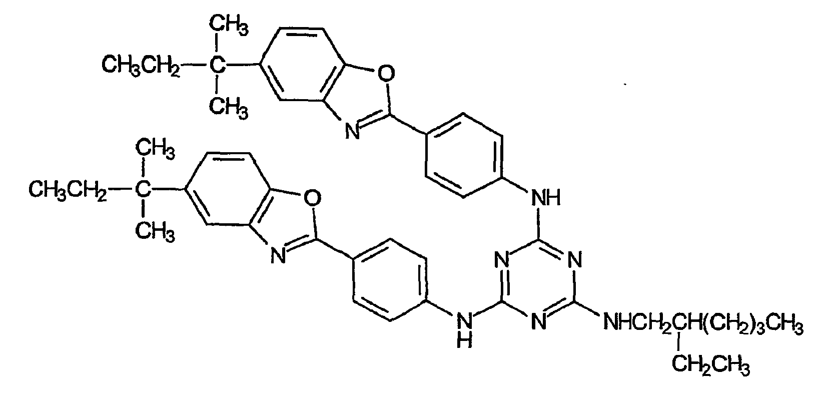 auszeichnet und bei 3V Sigma unter der Handelsbezeichnung Uvasorb® K2A erhältlich ist.

Das oder die Benzoxazol-Derivate liegen vorteilhaft in gelöster Form in den erfindungsgemäßen kosmetischen Zubereitungen vor. Es kann ggf. aber auch von Vorteil sein, wenn das oder die Benzoxazol-Derivate in pigmentärer, d. h. ungelöster Form - beispielsweise in Partikelgrößen von 10 nm bis zu 300 nm - vorliegen.

Vorteilhafte UV-Filtersubstanzen im Sinne der vorliegenden Erfindung sind ferner sogenannte Hydroxybenzophenone. Hydroxybenzophenone zeichnen sich durch die folgende Strukturformel aus: 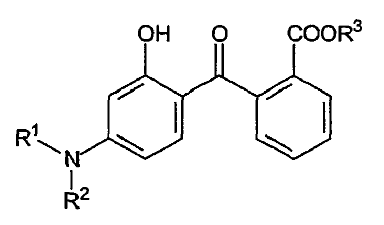 worin
- R¹ und R² unabhängig voneinander Wasserstoff, C₁-C₂₀-Alkyl, C₃-C₁₀-Cycloalkyl oder C₃-C₁₀-Cycloalkenyl bedeuten, wobei die Substituenten R¹ und R² gemeinsam mit dem Stickstoffatom, an das sie gebunden sind, einen 5- oder 6-Ring bilden können und
- R³ einen C₁-C₂₀-Alkyl Rest bedeutet.

Ein besonders vorteilhaftes Hydroxybenzophenon im Sinne der vorliegenden Erfindung ist der 2-(4'-Diethylamino-2'-hydoxybenzoyl)-benzoesäurehexylester (auch: Aminobenzophenon), welcher sich durch folgende Struktur auszeichnet: 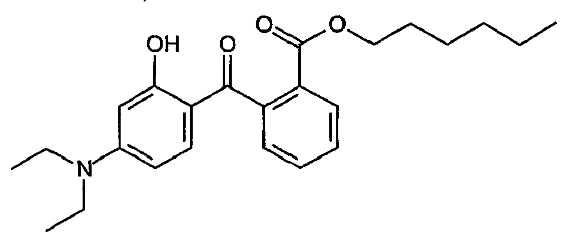 und unter dem Uvinul A Plus bei der Fa. BASF erhältlich ist.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Triazinderivate, wie z. B.
- 2,4-Bis-{[4-(2-Ethyl-hexyloxy)-2-hydroxy]-phenyl}-6-(4-methoxyphenyl)-1,3,5-triazin (INCI: Bis-Ethylhexyloxylphenol Methoxyphenyl Triazin), welches unter der Handelsbezeichnung Tinosorb® S bei der CIBA-Chemikalien GmbH erhältlich ist;
- Dioctylbutylamidotriazon (INCI: Diethylhexyl Butamido Triazone), welches unter der Handelsbezeichnung UVASORB HEB bei Sigma 3V erhältlich ist;
- 4,4',4"-(1,3,5-Triazin-2,4,6-triyltriimino)-tris-benzoesäure-tris(2-ethylhexylester), auch: 2,4,6-Tris-[anilino-(p-carbo-2'-ethyl-1'-hexyloxy)]-1,3,5-triazin (INCI: Ethylhexyl Triazone), welches von der BASF Aktiengesellschaft unter der Warenbezeichnung UVINUL® T 150 vertrieben wird;
- 2-[4,6-Bis(2,4-dimethylphenyl)-1,3,5-triazin-2-yl]-5-(octyloxy)phenol (CAS Nr.: 2725-22-6).

Ein vorteilhafter Breitbandfilter im Sinne der vorliegenden Erfindung ist auch das 2,2'-Methylen-bis-(6-(2H-benzotriazol-2-yl)-4-(1,1,3,3-tetramethylbutyl)-phenol) (INCI: Methylene Bis-Benztriazolyl Tetramethylbutylphenol), welches z. B. unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die weiteren UV-Filtersubstanzen können öllöslich oder wasserlöslich sein. Vorteilhafte öllösliche Filtersubstanzen sind z. B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- 2,4,6-Trianilino-(p-carbo-2'-ethyl-1'-hexyloxy)-1,3,5-triazin;
- Ester der Benzalmalonsäure, vorzugsweise 4-Methoxybenzalmalonsäuredi(2-ethylhexyl)ester;
- Ester der Zimtsäure, vorzugsweise 4-Methoxyzimtsäure(2-ethylhexyl)ester, 4-Methoxyzimtsäureisopentylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon sowie
- an Polymere gebundene UV-Filter.

### Vorteilhafte wasserlösliche Filtersubstanzen sind z. B.:

Sulfonsäure-Derivate des 3-Benzylidencamphers, wie z. B. 4-(2-Oxo-3-bomylidenmethyl)-benzolsulfonsäure, 2-Methyl-5-(2-oxo-3-bornylidenmethyl)sulfonsäure und deren Salze.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul^{®} N 539 T erhältlich ist.

Besonders vorteilhafte Zubereitungen im Sinne der vorliegenden Erfindung, die sich durch einen hohen bzw. sehr hohen UV-A-Schutz auszeichnen, enthalten neben der oder den erfindungsgemäßen Filtersubstanz(en) bevorzugt ferner weitere UV-A- und/oder Breitbandfilter, insbesondere Dibenzoylmethanderivate [beispielsweise das 4-(tert.-Butyl)-4'-methoxydibenzoylmethan] und/oder das 2,4-Bis-{[4-(2-Ethyl-hexyloxy)-2-hydroxy]-phenyl}-6-(4-methoxyphenyl)-1,3,5-triazin und/oder Phenylen-1,4-bis-(2-benzimidazyl)-3,3'-5,5'-tetrasulfonsäure-bis-natriumsalz jeweils einzeln oder in beliebigen Kombinationen miteinander.

Die Liste der genannten UV-Filter, die im Sinne der vorliegenden Erfindung eingesetzt werden können, soll selbstverständlich nicht limitierend sein.

Vorteilhaft enthalten die Zubereitungen gemäß der vorliegenden Erfindung die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen.

Die Zubereitungen im Sinne der vorliegenden Erfindung können ferner vorteilhaft Substanzen enthalten, die die Wasserfestigkeit der Produkte erhöhen, insbesondere wenn diese als Sonnenschutzprodukte verwendet werden sollen.

Vorteilhaft im Sinne der vorliegenden Erfindung sind PEG-45 Dodecylglykolcopolymer (INCI: PEG-45 Dodecyl Glycol Copolymer [y=z=11 und x=45]) und PEG-22 Dodecylglykolcopolymer (INCI: PEG-22 Dodecyl Glycol Copolymer [y=z=4,5 und x=22]) und Methoxy PEG-22 Dodecylglykolcopolymer (INCI: Methoxy PEG-22 Dodecyl Glycol Copolymer [y=7 und x=22 und R = CH₃]) zu verwenden, welche von der Firma AKZO Nobel erhältlich sind.

Vorteilhaft sind ferner beispielsweise wasserlösliche oder in Wasser dispergierbare Polyoxyethylen-Polyoxypropylen-Blockpolymere (CTFA-Bezeichnung: Polaxamere, CAS-Nr. 9003-11-6) mit folgender Struktur: 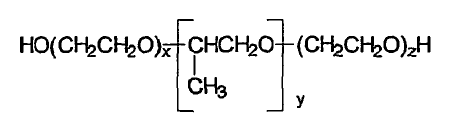 wobei x, y und z ganze Zahlen aus dem Bereich von 2 bis 130, insbesondere von 15 bis 100 darstellen und x und z gleich sind, aber unabhängig von y gewählt werden.

Unter diesen sind insbesondere Polaxamer 188 [mit x = 75, y = 30 und z = 75], welches unter der Handelsbezeichnung Lutrol F 68 (alt: Pluronic F 68) von der Firma BASF zu beziehen ist, das Polyxamer 185 [mit x = 19, y = 30 und z =19] (Lubrajel WA von ISP), das Polyxamer 235 [mit x = 27, y = 39 und z = 27] (Pluronic F 85 von BASF) und/oder das Polyxamer 238 [mit x = 97, y = 39 und z = 97] (Pluronic F 88 von BASF) vorteilhaft zu verwenden.

Weitere vorteilhafte Substanzen, welche zur Steigerung der Wasserfestigkeit beitragen können, aber in die Ölphase der Zubereitungen gemäß der vorliegende Erfindung eingearbeitet werden, sind bestimmte Wachskomponenten, wie acetyliertes Glykolstearat mit Tristearin (z. B. Unitwix von ISP mit der INCI: Acetylated Glycol Stearat and Tristearin), C₁₈₋₃₆ Fettsäuretriglycerid (z. B: Syncrowax HGLC von der Fa. Crode GmbH mit der INCI: C18-36 Acid Triglyceride) sowie die unter den Handelsbezeichnungen "Performa V 1608" (INCI: C30-38 Olefin/Isopropyl Maleate/MA Copolymer) und "Perfroma V 825" (synethisches Wachs) von New Phase Technologies erhältlichen Substanzen.

Es ist besonders vorteilhaft im Sinne der vorliegenden Erfindung die genannten Substanzen miteinander zu kombinieren, um die Wasserfestigkeit der Zubereitungen noch weiter zu verbessern.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Beispiele:

### 1

| **Selbstschäumende, schaumförmige O/W-Lotions und Cremes** | | | | | | | |
|---|---|---|---|---|---|---|---|
| | 1 | 2 | 3 | 4 | 5 | 6 | 7 |
| Palmitinsäure | | | | 1.00 | | | |
| Stearinsäure | 5.00 | 2.00 | 3.00 | | 4.00 | 4.00 | 1.00 |
| Cetylstearylalkohol | 5.50 | 0.50 | | | | 1.00 | 4.00 |
| Myristylalcohol | | 1.50 | | | | | 1.00 |
| Cetylalcohol | | | 8.50 | 2.00 | 2.00 | | |
| PEG-20 Stearat | | | 8.50 | | | | 1.00 |
| PEG-30 Stearat | 1.00 | | | | 2.00 | | |
| PEG-40 Stearat | | | | 5.50 | | | |
| PEG-100 Stearat | | 3.00 | | | | 1.00 | |
| Sorbitan Monostearat | | | | | 1.50 | | |
| Isopropylpalmitate | | | 2.00 | | | | |
| Cyclomethicon | 6.00 | | | | | | |
| Dimethicon | | | | 4.50 | | 0.50 | 0.50 |
| Caprylsäure/Caprinsäuretriglyceride | | | | | | | 2.00 |
| Octyl Isostearate | | | | | | | 5.00 |
| C12-15 Alkyl Benzoate | | | 5.00 | | | | |
| Dicaprylyl Carbonate | | | | 2.00 | 5.00 | | |
| Isohexadecan | | | 2.00 | | | | |
| Isoeikosan | 1.0 | | | | | | |
| Mineralöl / Paraffinöl | | 5.00 | | 4.50 | | 6.50 | 15.0 |
| Hydriertes Polyisobuten | | 15.0 | | | 5.00 | | |
| Polydecen | 6.0 | | | | | | |
| Magnesiumsilikat | 0.10 | | | | | | 0,10 |
| Magnesium-Aluminium- | | 0.20 | | | | 0.10 | |
| Silikat | | | | | | | |
| Hectorit | | | | 0,10 | | | |
| Quaternium-18-Hectorite | | | | | 0,10 | | |
| Carragen | 0,10 | | | | 0,10 | | |
| Cellulosegummi | | | | | | 0.10 | |
| Xanthan Gummi | | | | | 0,05 | | |
| Polyacrylsäure | | | | 0,10 | 0,10 | 0,10 | |
| Alkyl Acrylates Cosspolymere | | | | | | | 0,10 |
| Hydroxypropyl Starch Phosphate und deren Ester | | 0,25 | 0,50 | | | | |
| Stärke bzw.-Derivat (Aluminum Starch Octenylsuccinate etc.) | 0,20 | | | | | | |
| Anorg. Pigmente (wie Boron Nitride, Zeolithe, Kaolin, Siliciumoxid, Talkum, ilica Dimethyl Silylate etc.) | 0,90 | 1,25 | 1,0 | 1,25 | 0,60 | 0,50 | 0,25 |
| Citronensäure | 0.10 | | | | | | |
| Glycerin | 3.00 | 5.00 | 10.0 | 3.00 | 3,00 | 3,00 | 5.50 |
| Octylmethoxycinnamat | | | | | | | 4,00 |
| Butylmethoxydibenzoylmethan | | | | | | | 3,00 |
| Ethylhexyl Triazon | | | | | | | 3,00 |
| Glimmer | | | | 1.00 | | | |
| Eisenoxide | | | | 1.00 | | | |
| Titandioxid | | | | 4.50 | | | |
| Vitamin A Palmitate | | | | 0.10 | 0,20 | | |
| Vitamin-E-Acetat | | | | | 1,00 | 2,00 | |
| BHT | | | | | 0.02 | | 0.02 |
| Disodium EDTA | | | | | 0.10 | | 0.10 |
| Parfum | q.s. | q.s. | q.s. | q.s. | q.s. | q.s. | q.s. |
| Konservierung | q.s. | q.s. | q.s. | q.s. | q.s. | q.s. | q.s. |
| Natriumhydroxid | q.s. | q.s. | q.s. | q.s. | | | q.s. |
| Kaliumhydroxid | | | | | q.s | q.s | |
| Farbstoffe usw. | q.s | q.s. | q.s | q.s. | q.s | q.s. | q.s |
| Wasser | ad 100 | ad 100 | ad 100 | ad 100 | ad 100 | ad 100 | ad 100 |
| pH-Wert eingestellt auf | 6.0-7.5 | 5-6.5 | 6.2-7.0 | 5.0-6.4 | 5.3-6.7 | 6.0-7.5 | 6.0-7.5 |
| Volumen % der Grundlage | 60 | 85 | 75 | 40 | 90 | 80 | 95 |
| Volumen % Sauerstoff | 40 | 15 | 25 | 60 | 10 | 20 | 5 |

Vordispergierung des anorganischen Gelbildners und Quellung des Hydrokolloides unter Rühren in der Wasserphase. Vereinigung der auf 75 °C aufgeheizten Fettphase mit der auf 70 °C aufgeheizten Wasserphase. Zugabe der partikulären hydrophoben, hydrophobisierten Festkörpersubstanzen unter Rühren. Homogenisierung mittels einer Zahnkranzdispergiermaschine (Rotor-Stator-Prinzip) bei 65 °C. 45 min Rühren unter Vakuum und Kühlung. Zugabe der Additive bei 30 °C (Parfüm, Wirkstoffe). Homogenisierung mittels einer Zahnkranzdispergiermaschine (Rotor-Stator-Prinzip) bei 27 °C. Begasung der Emulsion bei 7-8 bar mittels eines dynamischen Schaumgenerators (z.B. von der Firma HANSA INDUSTRIE MIXER) mit Sauerstoff.

### 2

| **(schäumbare O/W-Creme)** | |
|---|---|
| **Emulsion** | **Gew.-%** |
| Stearinsäure | 4,00 |
| Cetylalkohol | 2,00 |
| PEG-30 Stearat ¹ | 2,00 |
| Sorbitan Monostearat² | 1,50 |
| Paraffinöl³ | 5,00 |
| Cyclomethicon ⁴ | 5,00 |
| Vitamin E Acetate | 1,00 |
| Retinylpalmitat | 0,20 |
| Glycerin | 3,00 |
| Alkohol kosmet. | 10,00 |
| BHT | 0,02 |
| Disodium EDTA | 0,10 |
| Parfüm, Konservierungsmittel, Farbstoffe | q.s. |
| Kaliumhydroxid | q.s. |
| Wasser | ad 100 |
| pH-Wert eingestellt auf 5,0-7,0 | |
| Zur Herstellung des Schaums werden 89 Vol.-% der Emulsion V mit 11 Vol.-% Sauerstoff aufgeschäumt^{a}. | |

| | |
|---|---|
| ¹ Myrj 51, ICI Surfactants, ²Glycomal S, Akzo Nobel, ³Pionier 2076, DEA Mineralöl ⁴Dow Corning Fluid 245, Dow Corning | |

### 3

| **(schäumbareO/W-Lotion):** | |
|---|---|
| **Emulsion** | **Gew.-%** |
| Stearinsäure | 4,00 |
| Cetylstearylalkohol | 1,00 |
| PEG-100 Stearat¹ | 1,00 |
| Octyldodecanol | 6,50 |
| Dimethicon³ | 2,50 |
| Vitamin E Acetat | 2,00 |
| Glycerin | 3,00 |
| Cetyl Ricinoleate | 2,00 |
| Parfüm, Konservierungsmittel, Farbstoffe | q.s. |
| usw. | |
| Natriumhydroxid | q.s. |
| Wasser | ad 100 |
| pH-Wert eingestellt auf 6,0-7,5 | |
| Zur Herstellung des Schaums werden 92 Vol.-% der Emulsion VI mit 8 Vol.-% eines Treibgasgemisches aus Propan und Butan (4Vol%) und Sauerstoff (4 Vol%) aufgeschäumt^{a}. | |

| | |
|---|---|
| ¹ Myrj 59p, ICI Surfactants, ³Wacker Silikonöl AK 50, Wacker | |

### 4

| **(schäumbare Sonnenschutz-Cremes):** | | | | |
|---|---|---|---|---|
| | **Emulsion 11** | | **Emulsion 12** | |
| | GEW.-% | Vol.-% | Gew.-% | Vol.-% |
| Stearinsäure | 5,00 | | 1,00 | |
| Cetylalkohol | 5,50 | | | |
| Cetylstearylalkohol | | | 2,00 | |
| PEG-40 Stearat | 8,50 | | | |
| PEG-20-Stearat | | | 1,00 | |
| CaprylsäurelCaprinsäuretriglyceride | 4,00 | | 2,00 | |
| C₁₂₋₁₅ Alkyl Benzoat | 10,00 | | 15,50 | |
| Cyclomethicon | 4,00 | | | |
| Dimethicon | | | 0,50 | |
| Octylisostearat | | | 5,00 | |
| Myristyl Myristat | | | 2,00 | |
| Ceresin | 1,50 | | | |
| Glycerin | 5,00 | | 10,00 | |
| UVASorb® K2A | 2,00 | | | |
| Uvinul A Plus® | 2,00 | | 1,50 | |
| Terephthaliden Dicamphersulfonsäure | 0,50 | | | |
| Drometrizol Trisiloxan | 1,50 | | | |
| Ethylhexylmethoxycinnamat | 5,00 | | 4,00 | |
| Ethylhexyltriazon | | | 3,00 | |
| Octocrylene | 5,00 | | | |
| Titandioxid Uvinul T 805 | 1,00 | | | |
| BHT | | | 0,02 | |
| Na₂H₂EDTA | 0,50 | | 0,10 | |
| Parfüm, Konservierungsmittel, | q.s. | | q.s. | |
| Farbstoffe, usw. | q.s. | | q.s. | |
| Kaliumhydroxid | q.s. | | q.s. | |
| Wasser | ad 100,00 | | ad 100,00 | |
| | pH-Wert eingestellt auf 6,5-7,5 | | pH-Wert eingestellt auf 5,0-6,0 | |
| **Emulsion 1** | | **70** | | |
| **Emulsion 2** | | | | **35** |
| **Gas (Sauerstoff)** | | **30** | | **65** |

| | | | | |
|---|---|---|---|---|
| Zur Herstellung des Schaums werden 85 Vol.-% der Emulsion 12, 13 mit Sauerstoff aufgeschäumt^{a}. | | | | |

### 5

| **O/W Emulsionen** | | | | | | | |
|---|---|---|---|---|---|---|---|
| | **13** | **14** | **15** | **16** | **17** | **18** | **19** |
| Glycerinmonostearat SE | 0,50 | 1,00 | 3,00 | | | 1,50 | |
| Glyceryl Stearat Citrat | 2,00 | | | 1,00 | 2,00 | | 2,50 |
| Stearinsäure | | 3,00 | 0,75 | 2,00 | | | |
| PEG-40 Stearat | 0,50 | | | | | 2,00 | |
| PEG-100 Stearat | | | 1,50 | | | | |
| Lauryl Methicon Copolyol | | | | 0,75 | | 0,50 | |
| Cetyl Phosphat | | | 0,75 | | 1,00 | | |
| Stearyl Alkohol | | | 3,00 | | | 2,00 | 0,50 |
| Cetyl Alkohol | 2,50 | 1,00 | | | 0,50 | | 2,00 |
| UVASorb® K2A | 1,00 | | | 4,00 | | 5,00 | |
| Uvinul® A Plus | 3,00 | 2,50 | 0,50 | 0,25 | 1,00 | 0,50 | 4,00 |
| Bis-Ethylhexyloxyphenol Methoxyphenyl Triazin | | 1,00 | | | | 1,00 | 0,50 |
| Disodium Phenyl Dibenzimidazol Tetrasulfonat | | | | 1,00 | | 2,00 | |
| Ethylhexyl Triazon | 2,00 | | | 2,00 | | 2,00 | |
| Diethylhexyl Butamido Triazon | | 2,00 | | | | | |
| Ethylhexyl Methoxycinnamat | | 3,50 | | 10,00 | | | |
| Octocrylen | | | | 5,00 | 9,00 | 7,50 | 2,50 |
| Methylen Bis-Benztriazolyl | | | 2,00 | | 3,00 | | |
| Tetramethylbutylphenol | | | | | | | |
| Ethylhexylsalicylat | | | 3,00 | | | | 5,00 |
| Drometrizol Trisiloxan | | | 0,50 | | | 1,00 | |
| Titandioxid T 805 | | 1,50 | | | 1,00 | 0,50 | |
| Titandioxid MT-100Z | 1,00 | | | 3,00 | 1,00 | | |
| C₁₂₋₁₅ Alkyl Benzoat | | 2,50 | | | | 7,00 | 5,00 |
| Dicaprylylether | | | 3,50 | | 2,00 | | |
| Butylenglycol Dicaprylat/ | 5,00 | | | 5,00 | 3,00 | | |
| Dicaprat | | | | | | | |
| Cetearyl Isononanoate | | 4,00 | | | | 2,00 | 2,00 |
| Dimethicon | | 0,50 | 1,00 | | 2,00 | | |
| Cyclomethicon | 2,00 | | | 4,50 | | | 0,50 |
| Dimethicon / Vinyl Dimethicon Crosspolymer | | 4,00 | | | | | 0,50 |
| PVP Hexadecen Copolymer | 0,50 | | | 0,50 | 1,00 | | 1,00 |
| Glycerin | 3,00 | 7,50 | | 7,50 | 5,00 | | 20,00 |
| Xanthangummi | 0,15 | | 0,05 | | | | 0,30 |
| Butylenglycol | 7,00 | 5,00 | | | | 7,00 | |
| Vitamin E Acetat | 0,50 | | 0,25 | 0,50 | 0,75 | | 1,00 |
| Dioic Acid | 0,25 | | | 0,20 | | 0,25 | |
| Fucogel® 1000 | | | 1,50 | | | 5,00 | |
| DMDM Hydantoin | | 0,60 | 0,40 | 0,20 | | | |
| Methylparaben | 0,15 | | 0,25 | | 0,50 | | |
| Phenoxyethanol | 1,00 | 0,40 | | 0,40 | 0,50 | | 0,60 |
| EDTA | | 0,20 | 0,35 | 0,50 | 0,02 | | 0,03 |
| Ethanol | | 2,00 | 1,50 | | 3,00 | 5,00 | 1,00 |
| Insekt Repellent 3535 | | | 5,00 | | | | |
| Parfüm | 0,20 | 0,20 | | | | 0,30 | 0,40 |
| Wasser | ad 100 | ad 100 | ad 100 | ad 100 | ad 100 | ad 100 | ad 100 |
| **Emulsion (Vol%)** | **80** | **85** | **50** | **70** | **95** | **60** | **30** |
| **Gas Sauerstoff (Vol%)** | **20** | **15** | **50** | **30** | **5** | **40** | **70** |

### 6

| **Dünnflüssige bis sprühbare W/O-Emulsionen (zur Verwendung als Spray oder Aerosol)** | | | | | |
|---|---|---|---|---|---|
| | **20** | **21** | **22** | **23** | **24** |
| Cetyl Dimethicon Copolyol | 4,00 | | | 2,50 | 3,00 |
| Polyglyceryl-2 Dipolyhydroxystearat | | | 3,00 | | 1,00 |
| Isostearyl Diglyceryl Succinat | | | 0,75 | | 0,30 |
| Lauryl Methicon Copolyol | | | | 2,00 | |
| Polysorbat-65 | | | 2,00 | | 1,50 |
| PEG-100 Stearat | | | | 1,20 | 0,70 |
| Cetearyl Sulfat | | | 0,25 | | 1,00 |
| Dimethicon | | 4,00 | | | 2,00 |
| Cyclomethicon | 12,00 | 20,00 | | 30,00 | 15,00 |
| UVASorb® K2A | | | | 0,50 | |
| Uvinul® A Plus | 3,50 | 2,00 | 0,50 | 4,00 | 0,25 |
| Bis-Ethylhexyloxyphenol Methoxyphenyl Triazin | 1,00 | | | 0,50 | |
| Dinatrium Phenyl Dibenzimidazol Tetrasulfonat | | 1,50 | | | 2,00 |
| Drometrizol Trisiloxan | | | | 1,00 | |
| Ethylhexyl Methoxycinnamat | 3,00 | 4,00 | | | 10,00 |
| Ethylheyl Salicylat | | | 5,00 | | 3,50 |
| Octocrylen | | 5,00 | | 4,00 | |
| Diethylhexyl Butamido Triazon | | 1,00 | | | 6,50 |
| Ethylhexyl Triazon | 3,00 | | | | 4,00 |
| Titandioxid MT-100 Z | | 0,50 | 1,00 | 1,50 | 0,50 |
| Zinkoxid Z-Cote | 2,00 | | | | 4,00 |
| Dicaprylyl Carbonat | 5,00 | | 15,00 | | 4,00 |
| Dihexyl Carbonat | | 10,00 | | | |
| C12-15 Alkyl Benzoat | 7,00 | | 10,00 | | |
| Mineral ÖI | 10,00 | | | | 6,00 |
| Cocoglyceride | | 2,00 | | 5,00 | |
| PVP Hexadecen Copolymer | | 0,75 | | | 0,40 |
| Glycerin | 5,00 | 12,50 | | 5,00 | 15,50 |
| Sorbitol | 5,00 | | 10,00 | | |
| α-Glucosylrutin | | | | | 0,15 |
| EDTA | | 0,15 | 0,03 | | 0,15 |
| Glycin Soja | 0,75 | | | 1,50 | |
| Magnesiumsulfat | 0,75 | 1,00 | | 0,45 | 1,00 |
| DMDM Hydantoin | | 0,05 | | | 0,10 |
| Phenoxyethanol | 1,00 | 0,75 | 0,50 | | 1,00 |
| Ethanol | 2,00 | | | 5,00 | 1,00 |
| Farbstoff, öllöslich | 0,02 | | | | |
| Parfüm | 0,30 | 0,45 | 0,35 | | 0,15 |
| Wasser | ad.100 | ad.100 | ad.100 | ad.100 | ad.100 |
| **Emulsion (Vol%)** | **80** | **85** | **50** | **70** | **95** |
| **Gas Sauerstoff (Vol%)** | **20** | **15** | **50** | **30** | **5** |

### 7

| **W/O-Emulsionen (Cremes & Lotions)** | | | | | |
|---|---|---|---|---|---|
| | **25** | **26** | **27** | **28** | **29** |
| Cetyldimethicon Copolyol | | | | 4,00 | |
| Polyglyceryl-2-dipolyhydroxystearat | 5,00 | 4,50 | | | 4,50 |
| PEG-30-Dipolyhydroxystearat | | | 5,00 | 2,00 | |
| UVASorb® K2A | | | | 2,50 | |
| Uvinul ® A Plus | 3,00 | 1,00 | 0,50 | 0,25 | 2,50 |
| Phenylbenzmidazol Sulfonsäure | | 4,00 | | 2,00 | 0,50 |
| Ethylhexyl Methoxycinnamat | | 8,00 | | 5,00 | 4,00 |
| Diethylhexyl Butamido Triazon | 3,00 | 1,00 | | | 3,00 |
| Ethylhexyl Triazon | | | 3,00 | 4,00 | |
| Octocrylen | 7,00 | | 8,00 | | 2,50 |
| Drometrizol Trisiloxan | | | 3,00 | | |
| Titandioxid Uvinul® T 805 | 2,00 | 1,00 | | | |
| Titandioxid MT-100 TV | | | 3,00 | | 2,00 |
| Zinkoxid Z-Cote® HP1 | 2,50 | | 6,00 | | |
| Mineralöl | | | 10,0 | | 8,00 |
| Cocoglyceride | 4,00 | 6,50 | | | |
| C12-15 Alkyl Benzoate | | | | 9,00 | |
| Dicaprylylether | 10,00 | | | | 7,00 |
| Butylenglycol Dicaprylat/Dicaprat | | | 2,00 | 8,00 | 4,00 |
| Cyclomethicon | 2,00 | | | | 2,00 |
| PVP Eicosene Copolymer | 0,50 | | | 1,50 | 1,00 |
| Trinatrium EDTA | 1,00 | | | 0,35 | |
| Ethylhexyloxyglycerin | | 0,30 | 1,00 | | 0,50 |
| Methylpropandiol | | | | | 7,50 |
| Glycerin | 5,00 | 7,50 | | 7,50 | 2,50 |
| Butylenglycol | | 2,50 | 15,00 | | |
| Glycin Soja | | 1,00 | 1,50 | | |
| MgSO₄ | 1,00 | 0,50 | | 0,50 | |
| Vitamin E | 0,50 | | 0,25 | | 1,00 |
| DMDM Hydantoin | | 0,60 | | 0,20 | |
| Methylparaben | 0,50 | | | 0,15 | |
| Phenoxyethanol | 0,50 | 0,40 | | 1,00 | 0,60 |
| Dihydroxiaceton | | | | 5,50 | |
| Ethanol | 3,00 | | 4,50 | | 1,00 |
| Parfüm | 0,20 | | 0,20 | | 0,20 |
| Wasser | ad 100 | ad 100 | ad 100 | ad 100 | ad 100 |
| **Emulsion (Vol%)** | **80** | **85** | **50** | **70** | **95** |
| **Gas Sauerstoff (Vol%)** | **20** | **15** | **50** | **30** | **5** |

### 8

| **Hydrodispersionen (zur Verwendung als Lotion oder Spray)** | | | | | |
|---|---|---|---|---|---|
| | **30** | **31** | **32** | **33** | **34** |
| PEG-40 Stearat | | 1,25 | | | |
| Cetyl Alkohol | | | | 2,00 | |
| Sodium Carbomer | | 0,20 | | 0,30 | |
| Acrylates/C10-30 Alkyl Acrylate Crosspolymer | | | 0,40 | 0,10 | 0,10 |
| Xanthan Gummi | 0,50 | 0,30 | 0,15 | | 0,50 |
| Dimethicon / Vinyl Dimethicon Crosspolymer | | | 5,00 | | 3,00 |
| UVASorb® K2A | | | | 3,50 | |
| Uvinul® A Plus | 0,25 | 3,50 | 0,50 | 2,00 | 1,50 |
| Bis-Ethylhexyloxyphenol Methoxypheny Triazin | | | 0,25 | | |
| Terephthaliden Dicampher Sulfonsäure | | 0,20 | | | 0,50 |
| Dinatrium Phenyl Dibenzimidazol Tetrasulfonat | 0,75 | | | | 1,00 |
| Ethylhexyl Methoxycinnamat | | | | 5,00 | 8,00 |
| Methylen Bis-Benzotriazolyl Tetramethylbutylphenol | | 1,00 | | | |
| Diethylhexyl Butamido Triazon | | | 2,00 | | |
| Ethylhexyl Triazon | 4,00 | | | 4,00 | |
| Octocrylen | | 4,00 | 10,00 | | 2,50 |
| Titandioxid MT-100 Z | 0,50 | | 2,00 | 3,00 | 1,00 |
| C₁₂₋₁₅ Alkyl Benzoate | 2,00 | 2,50 | | | |
| Butylenglycol Dicaprylat/Dicaprat | 4,00 | | | 6,00 | |
| Dicaprylyl Carbonat | | 3,00 | | | |
| Cyclomethicon | | | 7,50 | | |
| Lanolin | | | | 0,35 | |
| PVP Hexadecen Copolymer | 0,50 | | | 0,50 | 1,00 |
| Ethylhexyfoxyglycerin | | 0,50 | 1,00 | | 0,50 |
| Glycerin | 10,00 | 7,50 | | 5,00 | 15,00 |
| Glycin Soja | | 1,50 | 1,00 | | |
| Vitamin E Acetat | 0,50 | 0,20 | 0,25 | 0,75 | 1,00 |
| α - Glycosylrutin | | 0,30 | | 0,25 | |
| Trinatrium EDTA | | 0,30 | 0,10 | 0,20 | |
| Konkaben LMB ® | 0,20 | | | | 0,15 |
| Methylparaben | 0,50 | | | 0,15 | |
| Phenoxyethanol | 0,50 | | | 1,00 | 0,60 |
| Ethanol | 3,00 | 7,00 | 3,50 | | 1,00 |
| Parfüm, Farbstoffe | q.s. | q.s. | q.s. | q.s. | q.s. |
| Wasser | ad 100 | ad 100 | ad 100 | ad 100 | ad 100 |
| **Emulsion (Vol%)** | **80** | **85** | **50** | **70** | **95** |
| **Gas Sauerstoff (Vol%)** | **20** | **15** | **50** | **30** | **5** |

### 9

| **Ölgele** | | | | | |
|---|---|---|---|---|---|
| | **35** | **36** | **37** | **38** | **39** |
| Octyldodecanol | 9,00 | 9,00 | | | |
| Caprylic/Capric Triglycerid | 9,00 | | 6,00 | | |
| C₁₂₋₁₅- Alkyl Benzoate | | | | 5,00 | 8,00 |
| Butylenglycol Dicaprylat/Dicaprat | | 9,00 | | | 8,00 |
| Dicaprylylether | 9,00 | | | 4,00 | |
| Dicaprylylcarbonat | | 7,00 | | | |
| Ethyl Galaktomannan (N-Hance® AG 200) | 3,50 | | | | 4,00 |
| C₂₀₋₄₀ Fettsäuren + Polyethylen (Performacid®350) | | | | 2,00 | |
| Hydroxyoctacosanyl Hydroxystearat | 2,00 | | | | |
| Disteardimonium Hectorit | 1,00 | | | | 1,00 |
| Cyclomethicon | | | 15,00 | | 5,00 |
| UVASorb® K2A | | | | | 1,00 |
| Uvinul® A Plus | 1,00 | 3,50 | 2,75 | 2,00 | 0,50 |
| Ethylhexylmethoxycinnamat | 6,00 | | | 10,00 | 3,0 |
| Octocrylen | 3,50 | | 7,50 | 10,00 | |
| Ethylhexylsalicylat | | 3,50 | | | 4,00 |
| Ethylhexyl Triazon | | | 2,00 | | |
| Diethylhexyl Butamido Triazon | | 0,50 | | 3,00 | 4,0 |
| Phenoxyethanol | 0,50 | | | | |
| Parfüm, Farbstoff | q.s. | q.s. | q.s. | q.s. | q.s. |
| Mineralöl | ad. 100 | ad. 100 | ad.100 | | |
| Reisöl | | | | ad.100 | ad.100 |
| **Emulsion (Vol%)** | **80** | **85** | **50** | **70** | **95** |
| **Gas Sauerstoff (Vol%)** | **20** | **15** | **50** | **30** | **5** |

## Patentansprüche

1. Kosmetisches, nichttherapeutisches Verfahren zur Beseitigung degenerativer, alterungsassoziierter zellulärer Abläufe, wie sie in kutanen Alterungsprozess vorzufinden sind **dadurch gekennzeichnet, dass** eine Emulsion, enthaltend
1 bis 90 Vol.-% nicht molekular gebundenen freien Sauerstoff, bezogen auf das Gesamtvolumen der Emulsion, und
ein Emulgatorsystem, welches aus
a. mindestens einem Emulgator A, gewählt aus der Gruppe der ganz-, teil- oder nicht neutralisierten, verzweigten und/oder unverzweigten, gesättigten und/oder ungesättigten Fettsäuren mit einer Kettenlänge von 10 bis 40 Kohlenstoffatomen,
b. mindestens einem Emulgator B, gewählt aus der Gruppe der polyethoxylierten Fettsäureester mit einer Kettenlänge von 10 bis 40 Kohlenstoffatomen und mit einem Ethoxylierungsgrad von 5 bis 100 und
c. mindestens einem Coemulgator C, gewählt aus der Gruppe der gesättigten und/oder ungesättigten, verzweigten und/oder unverzweigten Fettalkohole mit einer Kettenlänge von 10 bis 40 Kohlenstoffatomen besteht,
und mindestens einmal täglich pulsartig auf die Haut aufgetragen wird, wobei die puls-artige Anwendung sich über maximal 1 Stunde erstreckt.

2. Verfahren nach Anspruch 1 **dadurch gekennzeichnet dass**, die Gesamtmenge an Sauerstoff aus dem Bereich 5 bis 50 Vol.-%, vorzugsweise 10 bis 40 Vol.-%, bezogen auf das Gesamtvolumen der Zubereitung, gewählt wird.

3. Verfahren nach mindestens einem der vorstehenden Ansprüche **dadurch gekennzeichnet, dass** der Sauerstoff aus Gasbläschen in einer selbstschäumenden, schaumförmigen, nachschäumenden oder schäumbaren Emulsion über einen Zeitraum von weniger als einer Stunde an die Haut abgegeben wird.

4. Verfahren nach mindestens einem der vorstehenden Ansprüche **dadurch gekennzeichnet, dass** Anwendung zwei- bis dreimal täglich wiederholt wird.

5. Verfahren nach mindestens einem der vorstehenden Ansprüche **dadurch gekennzeichnet, dass** das gestörte Erscheinungsbild der Haut entstanden ist durch Alters-bedingte und/oder intrinsische und/oder extrinsische Vorgänge.

6. Verfahren nach mindestens einem der vorstehenden Ansprüche **dadurch gekennzeichnet, dass** das gestörte Erscheinungsbild der Haut ausgeprägt ist durch:
• Falten und/oder Fältchen,
• Haut- und/oder Gewebeerschlaffung,
• Durchblutungsstörungen der Haut,
• Altersflecken,
• Sensible Haut
• Juckreiz,
• trockene Hautzustände,
• Pigmentstörungen, Uneven skin tone.

7. Verfahren nach mindestens einem der vorstehenden Ansprüche **dadurch gekennzeichnet, dass** die Zubereitung einen positiven Einfluss auf folgende Hauteigenschaften, wie
• Hautfarbe und Teint
• Hautgeschmeidigkeit
• Elastizität
• Hautstruktur
• Gleichgewichtszustand und Feuchtigkeitshaushalt, ausübt.

## Claims

1. Cosmetic non-therapeutic process for eliminating degenerative ageing-related cellular processes, as are found in the cutaneous ageing process, **characterized in that** an emulsion comprising
1 to 90% by volume of non-molecularly bonded free oxygen, based on the total volume of the emulsion, and
an emulsifier system which consists of
a. at least one emulsifier A selected from the group of completely neutralized, partially neutralized or unneutralized, branched and/or unbranched, saturated and/or unsaturated fatty acids with a chain length of from 10 to 40 carbon atoms,
b. at least one emulsifier selected from the group of polyethoxylated fatty acid esters with a chain length of from 10 to 40 carbon atoms and with a degree of ethoxylation of from 5 to 100 and
c. at least one coemulsifier C selected from the group of saturated and/or unsaturated, branched and/or unbranched fatty alcohols with a chain length of from 10 to 40 carbon atoms, is applied to the skin at least once daily in a pulse-like manner, where the pulse-like application lasts for a maximum of one hour.

2. Process according to Claim 1, **characterized in that** the total amount of oxygen is selected from the range 5 to 50% by volume, preferably 10 to 40% by volume, based on the total volume of the preparation.

3. Process according to at least one of the preceding claims, **characterized in that** the oxygen is released onto the skin from gas bubbles in a self-foaming, foam-like, after-foaming or foamable emulsion over a period of less than one hour.

4. Process according to at least one of the preceding claims, **characterized in that** application is repeated two to three times daily.

5. Process according to at least one of the preceding claims, **characterized in that** the impaired appearance of the skin has resulted through age-induced and/or intrinsic and/or extrinsic processes.

6. Process according to at least one of the preceding claims, **characterized in that** the impaired appearance of the skin is caused by:
• lines and/or wrinkles,
• slackening of the skin and/or tissue,
• circulation disturbances in the skin,
• age spots,
• sensitize skin,
• itchiness,
• dry skin conditions,
• pigment disorders, uneven skin tone.

7. Process according to at least one of the preceding claims, **characterized in that** the preparation exerts a positive influence on the following skin properties, such as
• skin colour and complexion
• skin suppleness
• elasticity
• skin texture
• state of equilibrium and moisture balance.

## Revendications

1. Procédé cosmétique, non thérapeutique, pour la suppression de processus cellulaires dégénératifs liés à l'âge, tels qu'ils apparaissent dans le processus de vieillissement cutané, **caractérisé en ce qu'**on applique sur la peau au moins une fois par jour par application successives, l'application par applications successives s'étendant sur 1 heure au maximum, une émulsion contenant
1 à 90 % en volume d'oxygène libre non en liaison moléculaire, par rapport au volume total de l'émulsion, et
un système d'émulsifiants qui consiste en
a. au moins un émulsifiant A, choisi dans le groupe des acides gras saturés et/ou insaturés, ramifiés et/ou non ramifiés, totalement, partiellement ou non neutralisés, ayant une longueur de chaîne de 10 à 40 atomes de carbone,
b. au moins un émulsifiant B, choisi dans le groupe des esters d'acides gras polyéthoxylés ayant une longueur de chaîne de 10 à 40 atomes de carbone et ayant un degré d'éthoxylation de 5 à 100 et
c. au moins un co-émulsifiant C, choisi dans le groupe des alcools gras saturés et/ou insaturés, ramifiés et/ou non ramifiés, ayant une longueur de chaîne de 10 à 40 atomes de carbone.

2. Procédé selon la revendication 1, **caractérisé en ce que** la quantité totale d'oxygène est choisie dans la plage allant de 5 à 50 % en volume, de préférence de 10 à 40 % en volume, par rapport au volume total de la préparation.

3. Procédé selon au moins l'une des revendications précédentes, **caractérisé en ce que** l'oxygène est libéré en un espace de temps de moins d'une heure sur la peau à partir de bulles de gaz dans une émulsion moussant d'elle-même, sous forme de mousse, post-moussante ou apte à être transformée en mousse.

4. Procédé selon au moins l'une des revendications précédentes, **caractérisé en ce que** l'application est répétée deux à trois fois par jour.

5. Procédé selon au moins l'une des revendications précédentes, **caractérisé en ce que** l'aspect perturbé de la peau résulte de processus dus à l'âge et/ou de processus intrinsèques et/ou extrinsèques.

6. Procédé selon au moins l'une des revendications précédentes, **caractérisé en ce que** l'aspect perturbé de la peau se manifeste par :
• des rides ou des ridules,
• des relâchements de la peau et/ou des tissus,
• des troubles de l'irrigation sanguine de la peau,
• des taches de sénescence,
• une peau sensible,
• un prurit,
• des états de sécheresse de la peau,
• des troubles de pigmentation, une coloration non uniforme de la peau (*uneven skin tone).*

7. Procédé selon au moins l'une des revendications précédentes, **caractérisé en ce que** la préparation a un effet positif sur les propriétés suivantes de la peau, à savoir
• couleur de la peau et teint
• souplesse de la peau
• élasticité
• texture de la peau
• état d'équilibre et état d'hydratation de la peau.
